# EUROPEAN PATENT SPECIFICATION

(11) **EP 1 086 245 B1**
(45) Date of publication and mention of the grant of the patent: **30.07.2003**
(21) Application number: 99928465.6
(22) Date of filing: 11.06.1999
(51) Int. Cl.: C12Q 1/68

(54) **MULTI-FLUORESCENT HAIRPIN ENERGY TRANSFER OLIGONUCLEOTIDES**
MEHRFACH FLUORESZIERENDE UND ENERGIE ÜBERTRAGENDE HAIRPIN-OLIGONUKLEOTIDE
OLIGONUCLEOTIDES DE TRANSFERT D'ENERGIE EN EPINGLE A CHEVEUX A MULTIFLUORESCENCE

(30) Priority: 12.06.1998 US 89119 P
(43) Date of publication of application: 28.03.2001
(73) Proprietor: Intergen Company, Purchase, NY 10577 (US)
(72) Inventor: NARDONE, Glenn, Oakton, VA 22124 (US)
(74) Representative: Wakerley, Helen Rachael
(86) International application number: US9912799
(87) International publication number: WO99064432

(56) References cited:
- EP-A- 0 566 751
- EP-A- 0 601 889
- WO-A-98/02449

## Description

### BACKGROUND OF THE INVENTION

### 1. Field of the Invention

The present invention is broadly concerned with an oligonucleotide probe useful for detecting multiple target nucleic acid sequences in a sample. More particularly, the present invention relates to an oligonucleotide probe labeled with a molecular energy transfer (MET) trio including an energy donor and two energy acceptors which makes possible the detection of the multiple targets using only one excitation wavelength of light.

### 2. Description of the Related Art

The MET phenomenon is a process by which energy is passed between a donor molecule and an acceptor molecule. Fluorescence resonance energy transfer (FRET), which involves at least one fluorophore, is a form of MET. A fluorophore is a compound that absorbs light at one wavelength, and emits light at different wavelength. A spectrofluorimeter is used to simultaneously emit light which excites the fluorophore, and detect light emitted by the fluorophore. In FRET, the fluorophore is a donor molecule which absorbs photons, and subsequently transfers this energy to an acceptor molecule. Donor and acceptor molecules that engage in MET or FRET are termed MET pairs or FRET pairs, respectively. Förster, 1949, Z. Naturforsch A4:321-327; Clegg, 1992, Methods In Enzymology 211:353-388.

When two fluorophores are in close proximity, and the emission spectrum of the first fluorophore overlaps the excitation spectrum of the second fluorophore, excitation of the first fluorophore causes it to emit light that is absorbed by the second fluorophore, which in turn causes the second fluorophore to emit light. As a result, the fluorescence of the first fluorophore is quenched, while the fluorescence of the second flourophore is enhanced. If the energy of the first fluorophore is transferred to a compound that is not a fluorophore, however, the fluorescence of the first fluorophore is quenched without subsequent emission of light by the non-fluorophore.

The FRET phenomenon has been exploited in methods for detecting nucleic acids. One of these methods is disclosed in U.S. patent No. 5,866,366. The '366 patent discloses a FRET-labeled hairpin oligonucleotide which is used as a probe in polymerase chain reaction (PCR) methods to detect target nucleic acid sequences. This oligonucleotide contains an energy donor and an energy acceptor constituting a FRET pair. The donor and acceptor are respectively situated on first and second nucleotide sequences of the oligonucleotide. These two nucleotide sequences are complementary to each other, and are therefore able to form a hairpin in the oligonucleotide.

If the first and second nucleotide sequences are annealed to each other, then the donor and acceptor are in close proximity. In this spatial arrangement, the acceptor absorbs the emission from the donor, and thereby quenches the signal from the donor. However, if the nucleotide sequences are not annealed to each other, then the donor and acceptor are separated, the acceptor can no longer absorb the emission from the donor, and the signal from the donor is not quenched.

Thus, if the oligonucleotide is incorporated into an amplification product during PCR, then the hairpin unfolds, resulting in the separation of the donor from the acceptor, and the consequent emission of an observable signal. However, if the oligonucleotide is not incorporated into a PCR amplification product, then the hairpin remains, and the emission from the donor is quenched by the acceptor. Detection of a signal after PCR therefore indicates the presence of the target.

Additionally, the FRET-labeled hairpin oligonucleotide described above may be used as a "molecular beacon" to detect a target nucleic acid sequence without incorporating it into a DNA molecule. The molecular-beacon technology is described in Tyagi et al., 1996, Nature Biotechnology 14:303-308, the entire contents of which are herein incorporated by reference. If the oligonucleotide hybridizes to a target, the hairpin unfolds, and a detectable signal is generated. If the oligonucleotide does not hybridize to the target, the hairpin remains, and the signal is quenched. Detection of a signal after hybridization therefore indicates the presence of the target.

Detection of more than one target nucleic acid molecule in a single sample using the methods disclosed in the '336 patent and Tyagi et al. requires a FRET-labeled hairpin oligonucleotide specific to each target molecule, wherein each donor emits a distinctive signal. Since each donor typically must be excited by a different wavelength of light, it is necessary to irradiate the sample with multiple wavelengths of light. A major drawback to this approach, however, is that it requires a spectrofluorimeter emitting a broad spectrum of light.

### SUMMARY OF THE INVENTION

Avoiding the aforementioned drawback, the present invention is directed to a MET-labeled oligonucleotide, as well as to the use of this oligonucleotide to detect multiple target nucleic acid sequences in a single sample. Pursuant to this invention, multiple targets can be detected by irradiating a sample, containing the targets, with a single excitation wavelength of light.

An oligonucleotide according to the present invention contains three nucleotide sequences: a first nucleotide sequence, a second nucleotide sequence at the 5' end of the first nucleotide sequence, and a third nucleotide sequence at the 5' end of the second nucleotide sequence. Additionally, the oligonucleotide contains a MET trio, *e.g*., a FRET trio, that includes an energy donor moiety and first and second energy acceptor moieties, where (i) the energy donor moiety is capable of emitting a quantum of energy and (ii) each of the first and second acceptor moieties is capable of absorbing a substantial amount of the quantum of energy. Preferably, the first acceptor moiety also is capable of emitting a quantum of energy.

The donor moiety is attached to a nucleotide of the first nucleotide sequence, the first acceptor moiety is attached to a nucleotide of the first nucleotide sequence, and the second acceptor moiety is attached to a nucleotide of the third nucleotide sequence, or, alternatively, the donor moiety is attached to a nucleotide of the third nucleotide sequence, the first acceptor moiety is attached to a nucleotide of the third nucleotide sequence, and the second acceptor moiety is attached to a nucleotide of the first nucleoxide sequence.

The oligonucleotide is capable of forming a hairpin containing a nucleotide of the first nucleotide sequence and a nucleotide of the third nucleotide sequence. If the donor moiety emits the quantum of energy, then the first acceptor moiety absorbs a substantial amount of the emitted quantum of energy if, preferably only if, the hairpin is not formed, and the second acceptor moiety absorbs a substantial amount of the emitted quantum of energy if, preferably only if, the hairpin is formed.

An oligonucleotide of this invention preferably comprises a fourth nucleotide sequence at the 3' end of the first nucleotide sequence. Ideally, the third nucleotide sequence is not complementary to the fourth nucleotide sequence, and the fourth nucleotide sequence is complementary to a nucleotide sequence flanking a target nucleotide sequence. *e.g*., a DNA sequence. The oligonucleotide may be included in a kit containing a polymerase. The oligonucleotide preferably comprises a deoxyribonucleotide.

Advantageously, the donor moiety and the first acceptor moiety each are fluorophores, while the second acceptor moiety is a quencher of light emitted by the donor moiety. The preferred donor moiety, first acceptor moiety, and second acceptor moiety are fluorescein, 6-carboxy-X-rhodamine (ROX), and 4-(4'-dimethylamino-phenylazo) benzoic acid (DABSYL), respectively.

Preferably, there is (are) 0 to 50 nucleotide(s) in between the nucleotide to which the donor moiety is attached and the nucleotide to which the first acceptor moiety is attached, and there is (are) 0 to 50 nucleotide(s) in between the nucleotide to which the donor moiety is attached and the nucleotide to which the second acceptor moiety is attached. More preferably, there are 5 to 10 nucleotides in between the nucleotide to which the donor moiety is attached and the nucleotide to which the first acceptor moiety is attached, and there are 5 to 10 nucleotides in between the nucleotide to which the donor moiety is attached and the nucleotide to which the second acceptor moiety is attached.

Advantageously, if the hairpin is formed, then the nucleotide to which the donor moiety is attached is the complement of the nucleotide to which the second acceptor moiety is attached, or there is (are) 0 to 5 nucleotide(s) in between the nucleotide to which the donor moiety is attached and the complement of the nucleotide to which the second acceptor moiety is attached.

A preferred oligonucleotide comprises or consists of the nucleotide sequence of SEQ ID NO:1, wherein fluorescein is attached to the nucleotide at position 1 of SEQ ID NO:1, ROX is attached to the nucleotide at position 21 of SEQ ID NO:1, and DABSYL is attached to the nucleotide at position 5 or 10 of SEQ ID NO:1.

One method of the present invention is a method for determining if a target nucleotide sequence is present in a sample comprising the following steps:

In step (a), a sample is contacted with an oligonucleotide containing a first nucleotide sequence, a second nucleotide sequence at the 5' end of the first nucleotide sequence, and a third nucleotide sequence at the 5' end of the second nucleotide sequence. The oligonucleotide also contains a MET trio including an energy donor moiety, and first and second energy acceptor moieties, wherein the donor moiety is capable of emitting a first quantum of energy, the first and second acceptor moieties are each capable of absorbing a substantial amount of the first quantum of energy, and the first acceptor moiety is capable of emitting a second quantum of energy. The preferred donor moiety, first acceptor moiety, and second acceptor moiety are fluorescein, ROX, and DABSYL, respectively.

The donor moiety is attached to a nucleotide of the first nucleotide sequence, the first acceptor moiety is attached to a nucleotide of the first nucleotide sequence, and the second acceptor moiety is attached to a nucleotide of the third nucleotide sequence, or, alternatively, the first donor moiety is attached to a nucleotide of the third nucleotide sequence, the first acceptor moiety is attached to a nucleotide of the third nucleotide sequence, and the second acceptor moiety is attached to a nucleotide of the first nucleotide sequence.

The oligonucleotide is capable of forming a hairpin containing a nucleotide of the first nucleotide sequence and a nucleotide of the third nucleotide sequence. If the donor moiety emits the first quantum of energy, then the first acceptor moiety absorbs a substantial amount of the emitted first quantum of energy if, preferably only if, the hairpin is not formed, and the second acceptor moiety absorbs a substantial amount of the emitted first quantum of energy if, preferably only if, the hairpin is formed.

In step (b), if the second quantum of energy is detected, then it is determined that the target nucleotide sequence is present in the sample, or if the second quantum of energy is not detected, then it is determined that the target nucleotide sequence is not present in the sample.

A second method of the present invention is a method for determining if a target nucleotide sequence is present in a sample comprising the following steps:

In step (a), a sample is contacted with an oligonucleotide containing a first nucleotide sequence, a second nucleotide sequence at the 5' end of the first nucleotide sequence, and a third nucleotide sequence at the 5' end of the second nucleotide sequence. The oligonucleotide also contains a MET trio including an energy donor moiety, and first and second energy acceptor moieties, wherein the donor moiety is capable of emitting a first quantum of energy, the first and second acceptor moieties are each capable of absorbing a substantial amount of the first quantum of energy, and the first acceptor moiety is capable of emitting a second quantum of energy. The preferred donor moiety, first acceptor moiety, and second acceptor moiety are fluorescein, ROX, and DABSYL, respectively.

The donor moiety is attached to a nucleotide of the first nucleotide sequence, the first acceptor moiety is attached to a nucleotide of the first nucleotide sequence, and the second acceptor moiety is attached to a nucleotide of the third nucleotide sequence, or, alternatively, the first donor moiety is attached to a nucleotide of the third nucleotide sequence, the first acceptor moiety is attached to a nucleotide of the third nucleotide sequence, and the second acceptor moiety is attached to a nucleotide of the first nucleotide sequence.

The oligonucleotide is capable of forming a hairpin containing a nucleotide of the first nucleotide sequence and a nucleotide of the third nucleotide sequence. If the donor moiety emits the first quantum of energy, then the first acceptor moiety absorbs a substantial amount of the emitted first quantum of energy if, preferably only if, the hairpin is not formed, and the second acceptor moiety absorbs a substantial amount of the emitted first quantum of energy if, preferably only if, the hairpin is formed.

In step (b), the oligonucleotide is incorporated, preferably using a polymerase, into a double-stranded nucleic acid if the target nucleotide sequence is present in the sample, thereby preventing the hairpin from forming.

In step (c), which is optional, an amplification reaction is conducted, resulting in the incorporation of the oligonucleotide into an amplification product if the target nucleotide sequence is present in the sample.

In step (d), if the second quantum of energy is detected, then it is determined that the target nucleotide sequence is present in the sample, or if the second quantum of energy is not detected, then it is determined that the target nucleotide sequence is not present in the sample.

A third method of the present invention is a method for detecting a target nucleotide sequence comprising the following steps:

In step (a), a first oligonucleotide is annealed to a nucleotide sequence flanking a target nucleotide sequence, wherein the first oligonucleotide contains a first nucleotide sequence, a second nucleotide sequence at the 5' end of the first nucleotide sequence, and a third nucleotide sequence at the 5' end of the second nucleotide sequence. The first oligonucleotide also contains a MET trio including an energy donor moiety, and first and second energy acceptor moieties, wherein the donor moiety is capable of emitting a first quantum of energy, and the first and second acceptor moieties are each capable of absorbing a substantial amount of the first quantum of energy. The preferred donor moiety, first acceptor moiety, and second acceptor moiety are fluorescein, ROX, and DABSYL, respectively.

The donor moiety is attached to a nucleotide of the first nucleotide sequence, the first acceptor moiety is attached to a nucleotide of the first nucleotide sequence, and the second acceptor moiety is attached to a nucleotide of the third nucleotide sequence, or, alternatively, the donor moiety is attached to a nucleotide of the third nucleotide sequence, the first acceptor moiety is attached to a nucleotide of the third nucleotide sequence, and the second acceptor moiety is attached to a nucleotide of the first nucleotide sequence.

The first oligonucleotide is capable of forming a hairpin containing a nucleotide of the first nucleotide sequence and a nucleotide of the third nucleotide sequence. If the donor moiety emits the first quantum of energy, then the first acceptor moiety absorbs a substantial amount of the emitted first quantum of energy if, preferably only if, the hairpin is not formed, and the second acceptor moiety absorbs a substantial amount of the emitted first quantum of energy if, preferably only if, the hairpin is formed.

The oligonucleotide advantageously comprises a fourth nucleotide sequence at the 3' end of the first nucleotide sequence. Ideally, the third nucleotide sequence is not complementary to the fourth nucleotide sequence, and the fourth nucleotide sequence is complementary to a nucleotide sequence flanking a target nucleotide sequence.

In step (b), the 3' end of the first oligonucleotide is extended using the target nucleotide sequence as a template to form an extended first strand, wherein the target nucleotide sequence is annealed to the extended first strand.

In step (c), the target nucleotide sequence is separated from the extended first strand.

In step (d), the second oligonucleotide is annealed to the extended first strand.

In step (e), the 3' end of the second oligonucleotide is extended using the extended first strand as a template to form an extended second strand, wherein the extended first strand is annealed to the extended second strand.

In step (f), which is optional, the extended first and second strands are amplified.

In step (g), a second quantum of energy emitted by the first acceptor moiety is detected to detect the target nucleotide sequence.

A variety of amplification methods can be used in step (f) to amplify the extended first and second strands (e.g., PCR amplification, strand displacement amplification, and cascade rolling circle amplification). The preferable method of amplification is PCR amplification comprising the following four steps:
In step (1), the extended first strand is separated from the extended second strand.
In step (2), the first oligonucleotide is annealed to the extended second strand, and the second oligonucleotide is annealed to the extended first strand.
In step (3), the 3' end of the first oligonucleotide is extended using the extended second strand as a template to form another extended first strand, wherein the extended second strand is annealed to the other extended first strand. Additionally, the 3' end of the second oligonucleotide is extended using the extended first strand as a template to form another extended second strand, wherein the extended first strand is annealed to the other extended second strand.
In step (4), steps (1), (2), and (3) are repeated for a finite number of times, wherein, in step (1), the extended first and second strands respectively are the extended first strand and the other extended second strand of step (3), or respectively are the other extended first strand and the extended second strand of step (3).

A fourth method of the present invention is a method for detecting a target nucleotide sequence comprising the following steps:

In step (a), a first oligonucleotide is annealed to a nucleotide sequence flanking a target nucleotide sequence, wherein the first oligonucleotide contains a first nucleotide sequence complementary to the nucleotide sequence flanking the target nucleotide sequence, and a second nucleotide sequence at the 5' end of the first nucleotide sequence.

In step (b), the 3' end of the first oligonucleotide is extended using the target nucleotide sequence as a template to form an extended first strand, wherein the target nucleotide sequence is annealed to the extended first strand.

In step (c), the target nucleotide sequence is separated from the extended first strand.

In step (d), a second oligonucleotide is annealed to the extended first strand.

In step (e), the 3' end of the second oligonucleotide is extended using the extended first strand as a template to form an extended second strand, wherein the extended first strand is annealed to the extended second strand.

In step (f), the extended first strand is separated from the extended second strand.

In step (g), a third oligonucleotide is annealed to the extended second strand, wherein the third oligonucleotide contains a first nucleotide sequence, a second nucleotide sequence at the 5' end of the first nucleotide sequence, a third nucleotide sequence at the 5' end of the second nucleotide sequence, and a fourth nucleotide sequence at the 3' end of the first nucleotide sequence. The third oligonucleotide also contains a MET trio comprising an energy donor moiety, and first and second energy acceptor moieties. The donor moiety is capable of emitting a quantum of energy, and the first and second acceptor moieties are each capable of absorbing a substantial amount of the quantum of energy. The preferred donor moiety, first acceptor moiety, and second acceptor moiety are fluorescein, ROX, and DABSYL, respectively.

The donor moiety is attached to a nucleotide of the first nucleotide sequence, the first acceptor moiety is attached to a nucleotide of the first nucleotide sequence, and the second acceptor moiety is attached to a nucleotide of the third nucleotide sequence, or, alternatively, the donor moiety is attached to a nucleotide of the third nucleotide sequence, the first acceptor moiety is attached to a nucleotide of the third nucleotide sequence, and the second acceptor moiety is attached to a nucleotide of the first nucleotide sequence.

The third oligonucleotide is capable of forming a hairpin containing a nucleotide of the first nucleotide sequence and a nucleotide of the third nucleotide sequence, and if the donor moiety emits the quantum of energy, then the first acceptor moiety absorbs a substantial amount of the emitted quantum of energy if, preferably only if, the hairpin is not formed, and the second acceptor moiety absorbs a substantial amount of the emitted quantum of energy if, preferably only if, the hairpin is formed.

The fourth nucleotide sequence is complementary to the complement of the second sequence of the first oligonucleotide. Ideally, the third nucleotide sequence is not complementary to the fourth nucleotide sequence, and the fourth nucleotide sequence is complementary to a nucleotide sequence flanking a target nucleotide sequence.

In step (h), the 3' end of the third oligonucleotide is extended using the extended second strand as a template to form a doubly extended first strand, wherein the doubly extended first strand is annealed to the extended second strand.

In step (i), the doubly extended first strand is separated from the extended labeled second strand.

In step (j), the second oligonucleotide is annealed to the doubly extended first strand.

In step (k), the 3' end of the second oligonucleotide is extended using the doubly extended first strand as a template to form a doubly extended second strand, wherein the doubly extended first strand is annealed to the doubly extended second strand.

In step (l), which is optional, the doubly extended first and second strands are amplified.

In step (m), a second quantum of energy emitted by the first acceptor moiety is detected to detect the target nucleotide sequence.

A variety of amplification methods can be used in step (1) to amplify the extended first and second strands (*e.g*., PCR amplification, strand displacement amplification, and cascade rolling circle amplification). The preferable method of amplification is PCR amplification comprising the following four steps:
In step (1), the doubly extended first strand is separated from the doubly extended second strand.
In step (2), the second oligonucleotide is annealed to the doubly extended first strand, and the third oligonucleotide is annealed to the doubly extended second strand.
In step (3), the 3' end of the second oligonucleotide is extended using the doubly extended first strand as a template to form another doubly extended second strand, wherein the doubly extended first strand is annealed to the other doubly extended second strand. Additionally, the 3' end of the third oligonucleotide is extended using the doubly extended second strand as a template to form another doubly extended first strand, wherein the doubly extended second strand is annealed to the other doubly extended first strand.
In step (4), steps (1), (2), and (3) are repeated for a finite number of times, wherein, in step (1), the doubly extended first and second strands respectively are the doubly extended first strand and the other doubly extended second strand of step (3), or respectively are the other doubly extended first strand and the doubly extended second strand of step (3).

A fifth method of the present invention is a method for determining if a first or second target nucleotide sequence is present in a sample comprising the following steps:
In step (a), the sample is contacted with first and second oligonucleotides. The first oligonucleotide contains a first nucleotide sequence, a second nucleotide sequence at the 5' end of the first nucleotide sequence, and a third nucleotide sequence at the 5' end of the second nucleotide sequence. The first oligonucleotide also contains a MET trio including a first energy donor moiety, and first and second energy acceptor moieties, wherein the first donor moiety is capable of emitting a first quantum of energy, and the first and second acceptor moieties are each capable of absorbing a substantial amount of the first quantum of energy.

The first donor moiety is attached to a nucleotide of the first nucleotide sequence, the first acceptor moiety is attached to a nucleotide of the first nucleotide sequence, and the second acceptor moiety is attached to a nucleotide of the third nucleotide sequence, or, alternatively, the first donor moiety is attached to a nucleotide of the third nucleotide sequence, the first acceptor moiety is attached to a nucleotide of the third nucleotide sequence, and the second acceptor moiety is attached to a nucleotide of the first nucleotide sequence.

The first oligonucleotide is capable of forming a first hairpin containing a nucleotide of the first nucleotide sequence and a nucleotide of the third nucleotide sequence. If the first donor moiety emits the first quantum of energy, then the first acceptor moiety absorbs a substantial amount of the emitted first quantum of energy if, preferably only if, the first hairpin is not formed, and the second acceptor moiety absorbs a substantial amount of the emitted first quantum of energy if, preferably only if, the first hairpin is formed.

The second oligonucleotide contains a fourth nucleotide sequence, a fifth nucleotide sequence at the 5' end of the fourth nucleotide sequence, a sixth nucleotide sequence at the 5' end of the fifth nucleotide sequence. The second oligonucleotide also contains a MET pair including a second energy donor moiety and a third energy acceptor moiety, wherein the second donor moiety is capable of emitting a second quantum of energy, and the third acceptor moiety is capable of absorbing a substantial amount of the second quantum of energy.

The second donor moiety is attached to a nucleotide of the fourth nucleotide sequence and the third acceptor moiety is attached to a nucleotide of the sixth nucleotide sequence, or, alternatively, the second donor moiety is attached to a nucleotide of the sixth nucleotide sequence and the third acceptor moiety is attached to a nucleotide of the fourth nucleotide sequence.

The second oligonucleotide is capable of forming a second hairpin containing a nucleotide of the fourth nucleotide sequence and a nucleotide of the sixth nucleotide sequence. If the second donor moiety emits the second quantum of energy, then the third acceptor moiety absorbs a substantial amount of the emitted second quantum of energy if, preferably only if, the second hairpin is formed.

The preferred first and second donor moieties each are fluorescein, the preferred first acceptor moiety is ROX, and the preferred second and third acceptor moieties each are DABSYL. Ideally, the first oligonucleotide comprises a seventh nucleotide sequence at the 3' end of the first nucleotide sequence, the third nucleotide sequence is not complementary to the seventh nucleotide sequence, the seventh nucleotide sequence is complementary to a nucleotide sequence flanking the first target nucleotide sequence, the second oligonucleotide further comprises an eighth nucleotide sequence at the 3' end of the fourth nucleotide sequence, the sixth nucleotide sequence is not complementary to the eighth nucleotide sequence, and the eighth nucleotide sequence is complementary to a nucleotide sequence flanking the second target nucleotide sequence.

In step (b) the first oligonucleotide is incorporated, preferably using a polymerase, into a first double-stranded nucleic acid if the first target nucleotide sequence is present in the sample, thereby preventing the first hairpin from forming. Additionally, the second oligonucleotide is incorporated, preferably using a polymerase, into a second double-stranded nucleic acid if the second target nucleotide sequence is present in the sample, thereby preventing the second hairpin from forming.

In step (c), which is optional, a first amplification reaction is conducted, thereby incorporating the first oligonucleotide into a first amplification product if the first target nucleotide sequence is present in the sample. Additionally, a second amplification reaction is conducted, thereby incorporating the second oligonucleotide into a second amplification product if the second target nucleotide sequence is present in the sample.

In step (d), it is determined that the first target nucleotide sequence is present in the sample if a third quantum of energy emitted by the first acceptor moiety is detected, or it is determined that the first target nucleotide sequence is not present in the sample if the third quantum of energy is not detected. Additionally, it is determined that the second target nucleotide sequence is present in the sample if a fourth quantum of energy emitted by the third acceptor moiety is detected, or it is determined that the second target nucleotide sequence is not present in the sample if the fourth quantum of energy is not detected.

Other objects, features, and advantages of the present invention will become apparent from the following detailed description. It should be understood, however, that the description and the specific examples, while indicating preferred embodiments of the invention, are given by way of illustration only, because various changes and modifications within the spirit and scope of the invention will become apparent to those skilled in the art from this detailed description.

### DETAILED DESCRIPTION OF THE PREFERRED EMBODIMENT

As explained above, the oligonucleotide of the present invention contains two nucleotide sequences that are capable of hybridizing to each other to form a hairpin. One nucleotide sequence contains an energy donor and first energy acceptor, while the other nucleotide sequence contains a second energy acceptor that is able to quench the signal generated by the donor. Thus, the present oligonucleotide contains a MET trio. In a preferred embodiment, the donor and first acceptor are fluorophores, the emission spectrum of the donor fluorophore overlaps the excitation spectrum of the acceptor fluorophore (*i.e*., the first acceptor), and the second acceptor is a quencher of the signal emitted by the donor fluorophore.

In accordance with the present invention, the donor fluorophore and the quencher are positioned on the oligonucleotide so that, if the hairpin is formed, then the donor fluorophore is in close proximity to the quencher, and energy emitted by the donor fluorophore is absorbed by the quencher, resulting in quenching of the signal from the donor fluorophore. If the hairpin is not formed, however, and the quencher consequently does not absorb the energy emitted by the donor, then the emitted energy is absorbed by the acceptor fluorophore, and is subsequently emitted by the acceptor fluorophore as a detectable signal.

Pursuant to the present invention, detection of a target nucleic acid sequence in a sample occurs in one of at least two ways. First, the oligonucleotide may be incorporated into an amplification product which contains the target sequence. Second, the oligonucleotide may be hybridized to the target sequence. In a preferred embodiment, irradiation of the sample, using a spectrofluorimeter after such incorporation or hybridization, causes the acceptor fluorophore to emit a signal which is detected by the spectrofluorimeter. Thus, signal detection indicates that the target is present, while the absence of a signal indicates that the target is not present.

A sample is assayed for the presence of two targets by using the oligonucleotide described above in conjunction with another oligonucleotide. The other oligonucleotide also contains two nucleotide sequences capable of forming a hairpin, along with a donor fluorophore and a quencher positioned, with respect to the hairpin, as in the present oligonucleotide. The donors of the present oligonucleotide and the other oligonucleotide are excited by the same or approximately the same wavelength of light. However, the other oligonucleotide does not contain the acceptor fluorophore, and therefore contains a MET pair rather than a MET trio. The present oligonucleotide and other oligonucleotide are specific to the first and second targets, respectively.

If the sample contains both the first and second targets, then a hairpin is prevented from forming in both the present oligonucleotide and the other oligonucleotide after the amplification or hybridization reactions are conducted. Subsequent irradiation of the sample with a single wavelength of light that excites the donor fluorophore of the present oligonucleotide and the other oligonucleotide, but not the acceptor fluorophore of the present oligonucleotide, causes two distinctive signals to be generated. The first signal is emitted by the acceptor moiety of the present oligonucleotide, while the second signal is emitted by the donor fluorophore of the other oligonucleotide. Thus, the first target is detected by observing the first signal, while the second target is detected by observing the second signal. Two targets therefore can be detected by irradiating the sample with only one wavelength of light.

The invention is further described by reference to the examples below, with are set forth by illustration only.

### Example 1

### Spectroscopic analysis of model FRET-labeled hairpin oligonucleotides each containing a donor fluorophore, an acceptor fluorophore, and a quencher

A total of seven oligonucleotides were synthesized. All of these oligonucleotides have the following nucleotide sequence: 5'-ACCGATGCGTTGAGCATCGGTGAAGGTC-GGAGTCAACGGATT-3' (SEQ ID NO:1). All of the oligonucleotides contain a fluorescein moiety (*i.e.*, the first fluorophore) attached to the nucleotide at position 1 and a DABSYL moiety attached to the nucleotide at position 21. Oligonucleotides 1 through 4 each contain a second fluorophore attached to the nucleotide at position 5, while oligonucleotides 5 through 7 each contain a second fluorophore attached to the nucleotide at position 10. The second fluorophore in oligonucleotides 1 and 5 is N,N,N',N'-tetramethyl-6-carboxyrhodamine (TAMRA), in oligonucleotides 3 and 6 is ROX, in oligonucleotides 4 and 7 is Texas Red, and in oligonucleotide 2 is Bodipy 564/570.

The fluorescein and DABSYL moieties were incorporated into each oligonucleotide during their synthesis using a dA-fluorescein phosphoramidite and a dU-DABSYL phosphoroamidite, respectively. Additionally, a hexylamine moiety was attached to the carbon atom at the 5 position of the base moiety of the nucleotides at position 5 of oligonucleotides 1 through 4, and at position 10 of oligonucleotides 5 through 7. This hexylamine moiety was incorporated into each oligonucleotide during their synthesis using a dU-hexylamine phosphoramidite.

After the oligonucleotides were synthesized as describe above, each oligonucleotide was deprotected, desalted, and purified by reverse-phase high-performance liquid chromatography using C18 silica and triethylamine acetate-acetonitrile gradients. The appropriate fractions were subsequently pooled, precipitated using acetone, dried under vacuum, dissolved, and desalted.

The hexylamine residue of each oligonucleotide was then labeled with the appropriate fluorophore containing an N-succinimidyl ester or an isothiocyanate moiety in a solution of sodium bicarbonate buffer, pH 9, 20% dimethyl sulfoxide at room temperature for approximately 20 hours. Each oligonucleotide was subsequently precipitated using acetone, dried under vacuum, dissolved, and desalted.

For spectroscopic analysis of each of the oligonucleotides, four to five picomoles of oligonucleotide were dissolved in a volume of 0.6 ml. Each oligonucleotide solution was analyzed using a Shimadzu RFU 5000 analytical spectrofluorimeter. To induce the closed confirmation, in which the oligonucleotide contains a hairpin, the oligonucleotide was dissolved in annealing buffer containing 10 mM Tris-HCl, pH 8, 3 mM MgCl₂, and 50 mM NaCl. The open confirmation, in which the hairpin is absent, was induced by adding a molar excess of a complementary, unlabeled oligonucleotide to each oligonucleotide in annealing buffer, heating the solution at 90°C for three minutes, and cooling the solution to room temperature.

The fluorescein moiety of each oligonucleotide in the open and closed conformations was excited by irradiation. Table 1 below demonstrates that such irradiation results in a substantial light emission from the second fluorophore of each oligonucleotide in the open conformation. In the closed conformation, however, each oligonucleotide shows little second-fluorophore emission at fluorescein excitation wavelengths.

**Table 1**

| oligonucleotide | second fluorophore | excitation wavelength (nm)¹ / emission wavelength (nm)² | fluorescence per picomole in closed conformation (relative fluoresence units) | fluorescence per picomole in open conformation (relative fluoresence units) |
|---|---|---|---|---|
| 1 | TAMRA | 495/588 | 6 | 28 |
| 2 | Bodipy 564/570 | 495/598 | 7 | 48 |
| 3 | ROX | 495/605 | 3 | 34 |
| 4 | Texas Red | 495/620 | 5 | 26 |
| 5 | TAMRA | 495/588 | 7 | 30 |
| 6 | ROX | 495/605 | 4 | 18 |
| 7 | Texas Red | 495/620 | 4 | 30 |

| | | | | |
|---|---|---|---|---|
| ¹ Wavelength of light exciting fluorescein. | | | | |
| ² Wavelength of light emitted from the second fluorophore; the wavelength of each second-fluorophore emission is substantially different from the peak wavelength of fluorescein emission (516 nm). | | | | |

### Example 2

### Detection of two target nucleic acid sequences in PCR amplification using a hairpin oligonucleotide containing a FRET trio and a hairpin oligonucleotide containing a FRET pair

A sample is assayed for the presence of mRNA encoded by a prostate-specific antigen (PSA) gene. As a control to insure that a PCR reaction can be conducted in the sample, a quantity of cDNA specific to the glyceraldehyde-3-phosphate dehydrogenase (gapDH) gene is added to the sample.

The hairpin oligonucleotides are used as forward primers during PCR. The nucleotide sequence of the forward primer specific to the gapDH cDNA is 5'-AGCGATGCGTTCGAGCATCGCTGAAGGTCGGAGTCAACGGATT-3' (SEQ ID NO:2), wherein the nucleotides at positions 1 and 22 are labeled with fluorescein and DABSYL, respectively, and the nucleotide at position 5 or 10 is labeled with TAMRA. The nucleotide sequence of the hairpin primer specific to the PSA mRNA is 5'-AGCGATGCGTTCGAGCATCGCTGAAGGTGACCAAGTTCAT-3' (SEQ ID NO:3), wherein the nucleotides at positions 1 and 22 are labeled with fluorescein and DABSYL, respectively. The nucleotide sequences of the reverse primers specific to the gapDH cDNA and the PSA mRNA are 5'-GGATCTCGCTCCTGGAAGATGGT-3' (SEQ ID NO:4), and 5'-GGTGTACAGGGAAGGCCTTTCGGGAC-3' (SEQ ID NO:5), respectively.

Each PCR reaction sample contains, in a 0.2 ml PCR reaction tube, 10 mM Tris-HCl, pH 8.3, 50 mM KCI, 1.5 mM MgCl₂, 250 µm dNTPs, 0.25 µm of each forward primer, 0.25 µm of each reverse primer, gapDH cDNA, and 2 units of Taq polymerase in 50 µl. A control sample additionally contains PSA mRNA, which, for example, is produced by LNCaP tissue-culture cells available from the American Type Culture Collection, Manassas, VA.

Each reaction tube is placed in a thermocycler such as an ABI 7700 real-time instrument, or a Perkin Elmer 9600 or 9700. The cycle program is as follows: [94°C, 4 min.]-[94°C, 15 sec.; 55°C, 30 sec.; 72°C, 1 min] for 35 cycles - [72°C, 5 min] - [4°C, hold]. The progress of gapDH cDNA amplification is followed directly by observing the emission of light having a wavelength of 580 nm (*i.e*., the emission from TAMRA), while the progress of PSA mRNA amplification is followed directly by observing the emission of light having a wavelength of 530 nm (the emission from fluorescein). End-point analysis is effected by means of a fluorescent plate reader, such as a Wallac Victor equipped with filters that allow detection of 530-nm and 580-nm light.

## Claims

1. An oligonucleotide containing:
(a) a first nucleotide sequence;
(b) a second nucleotide sequence at the 5' end of the first nucleotide sequence;
(c) a third nucleotide sequence at the 5' end of the second nucleotide sequence; and
(d) a molecular energy transfer trio including an energy donor moiety, and first and second energy acceptor moieties,
wherein,
the energy donor moiety is capable of emitting a quantum of energy, and the first and second acceptor moieties are each capable of absorbing a substantial amount of the quantum of energy,
the donor moiety is attached to a nucleotide of the first nucleotide sequence, the first acceptor moiety is attached to a nucleotide of the first nucleotide sequence, and the second acceptor moiety is attached to a nucleotide of the third nucleotide sequence; or the donor moiety is attached to a nucleotide of the third nucleotide sequence, the first acceptor moiety is attached to a nucleotide of the third nucleotide sequence, and the second acceptor moiety is attached to a nucleotide of the first nucleotide sequence,
the oligonucleotide is capable of forming a hairpin containing a nucleotide of the first nucleotide sequence and a nucleotide of the third nucleotide sequence, and
if the donor moiety emits the quantum of energy, then:
(1) the first acceptor moiety absorbs a substantial amount of the emitted quantum of energy if the hairpin is not formed, and
(2) the second acceptor moiety absorbs a substantial amount of the emitted quantum of energy if the hairpin is formed.

2. The oligonucleotide of claim 1, wherein the first acceptor moiety absorbs the substantial amount of the emitted quantum of energy only if the hairpin is not formed.

3. The oligonucleotide of claim 1, wherein the second acceptor moiety absorbs the substantial amount of the emitted quantum of energy only if the hairpin is formed.

4. The oligonucleotide of any of claims 1 to 3, comprising a deoxyribonucleotide.

5. The oligonucleotide of any of claims 1 to 4, further comprising a fourth nucleotide sequence at the 3' end of the first nucleotide sequence.

6. The oligonucleotide of claim 5, wherein the third nucleotide sequence is not complementary to the fourth nucleotide sequence.

7. The oligonucleotide of claim 5, wherein the fourth nucleotide sequence is complementary to a nucleotide sequence flanking a target nucleotide sequence.

8. The oligonucleotide of claim 7, wherein the target nucleotide sequence is DNA.

9. The oligonucleotide of any preceding claim, wherein the donor moiety is a fluorophore.

10. The oligonucleotide of claim 9, wherein the second acceptor moiety is a quencher of light emitted by the fluorophore.

11. The oligonucleotide of any preceding claim, wherein the first acceptor moiety is capable of emitting another, second quantum of energy.

12. The oligonucleotide of any preceding claim, wherein the first acceptor moiety is a fluorophore.

13. The oligonucleotide according to any preceding claim, wherein the donor moiety is fluorescein.

14. The oligonucleotide according to any preceding claim, wherein the first acceptor moiety is ROX.

15. The oligonucleotide of any preceding claim, wherein the second acceptor moiety is DABSYL.

16. The oligonucleotide of any preceding claim, wherein there is(are) 0 to 50 nucleotide(s) in between the nucleotide to which the donor moiety is attached and the nucleotide to which the first acceptor moiety is attached.

17. The oligonucleotide of claim 16, wherein there are 5 to 10 nucleotides in between the nucleotide to which the donor moiety is attached and the nucleotide to which the first acceptor moiety is attached.

18. The oligonucleotide of any preceding claim, wherein there is(are) 0 to 50 nucleotide(s) in between the nucleotide to which the donor moiety is attached and the nucleotide to which the second acceptor moiety is attached.

19. The oligonucleotide of claim 18, wherein there are 5 to 10 nucleotides in between the nucleotide to which the donor moiety is attached and the nucleotide to which the second acceptor moiety is attached.

20. The oligonucleotide of any preceding claim, wherein, if the hairpin is formed, then the nucleotide to which the donor moiety is attached is the complement of the nucleotide to which the second acceptor moiety is attached.

21. The oligonucleotide of any preceding claim, wherein, if the hairpin is formed, then there is(are) 0 to 5 nucleotide(s) in between the nucleotide to which the donor moiety is attached and the complement of the nucleotide to which the second acceptor moiety is attached.

22. An oligonucleotide comprising the nucleotide sequence of SEQ ID NO:1, wherein fluorescein is attached to the nucleotide at position 1 of SEQ ID NO:1, ROX is attached to the nucleotide at position 21 of SEQ ID NO:1, and DABSYL is attached to the nucleotide at position 5 or 10 of SEQ ID NO:1.

23. The oligonucleotide of claim 22 consisting of the nucleotide sequence of SEQ ID NO:1.

24. A kit comprising the oligonucleotide of any preceding claim and a polymerase.

25. The kit of claim 24, wherein the polymerase is a DNA polymerase.

26. A method for determining if a target nucleotide sequence is present in a sample comprising:
(a) contacting the sample with an oligonucleotide of any of claims 11 to 21 (when dependent on claim 11), 22 or 23; and
(b) if the second quantum of energy is detected, then determining that the target nucleotide sequence is present in the sample; or if the second quantum of energy is not detected, then determining that the target nucleotide sequence is not present in the sample.

27. A method for determining if a target nucleotide sequence is present in a sample comprising:
(a) contacting the sample with an oligonucleotide according to any of claims 11 to 21 (when dependent on claim 11), 22 or 23;
(b) incorporating the oligonucleotide into a double-stranded nucleic acid if the target nucleotide sequence is present in the sample, thereby preventing the hairpin from forming;
(c) optionally conducting an amplification reaction, thereby incorporating the oligonucleotide into an amplification product if the target nucleotide sequence is present in the sample; and
(d) if the second quantum of energy is detected, then determining that the target nucleotide sequence is present in the sample; or if the second quantum of energy is not detected, then determining that the target nucleotide sequence is not present in the sample.

28. The method of claim 27, wherein in step (b), the oligonucleotide is incorporated into the double-stranded nucleic acid using a polymerase.

29. A method for detecting a target nucleotide sequence comprising:
(a) annealing a first oligonucleotide to a nucleotide sequence flanking a target nucleotide sequence, wherein the first oligonucleotide is one according to any of claims 11 to 23;
(b) extending the 3' end of the first oligonucleotide using the target nucleotide sequence as a template to form an extended first strand, wherein the target nucleotide sequence is annealed to the extended first strand;
(c) separating the target nucleotide sequence from the extended first strand;
(d) annealing a second oligonucleotide to the extended first strand;
(e) extending the 3' end of the second oligonucleotide using the extended first strand as a template to form an extended second strand, wherein the extended first strand is annealed to the extended second strand;
(f) optionally amplifying the extended first and second strands; and
(g) detecting a second quantum of energy emitted by the first acceptor moiety to detect the target nucleotide sequence.

30. The method according to claim 29, wherein step (f) comprises:
(1) separating the extended first strand from the extended second strand;
(2) annealing the first oligonucleotide to the extended second strand, and annealing the second oligonucleotide to the extended first strand;
(3) extending the 3' end of the first oligonucleotide using the extended second strand as a template to form another extended first strand, wherein the extended second strand is annealed to the other extended first strand; and extending the 3' end of the second oligonucleotide using the extended first strand as a template to form another extended second strand, wherein the extended first strand is annealed to the other extended second strand; and
(4) repeating steps (1), (2) and (3) for a finite number of times, wherein, in step (1), the extended first and second strands respectively are the extended first strand and the other extended second strand of step (3), or respectively are the other extended first strand and the extended second strand of step (3).

31. A method for detecting a target nucleotide sequence comprising:
(a) annealing a first oligonucleotide to a nucleotide sequence flanking a target nucleotide sequence, wherein the first oligonucleotide contains:
(1)a first nucleotide sequence complementary to the nucleotide sequence flanking the target nucleotide sequence, and
(2) a second nucleotide sequence at the 5' end of the first nucleotide sequence.
(b) extending the 3' end of the first oligonucleotide using the target nucleotide sequence as a template to form an extended first strand, wherein the target nucleotide sequence is annealed to the extended first strand;
(c) separating the target nucleotide sequence from the extended first strand;
(d) annealing a second oligonucleotide to the extended first strand;
(e) extending the 3' end of the second oligonucleotide using the extended first strand as a template to form an extended second strand, wherein the extended first strand is annealed to the extended second strand;
(f) separating the extended first strand from the extended second strand;
(g) annealing a third oligonucleotide to the extended second strand, wherein the third oligonucleotide contains:
(1) a first nucleotide sequence,
(2) a second nucleotide sequence at the 5' end of the first nucleotide sequence,
(3) a third nucleotide sequence at the 5' end of the second nucleotide sequence,
(4) a fourth nucleotide sequence at the 3' end of the first nucleotide sequence, and
(5) a molecular energy transfer trio comprising an energy donor moiety, and first and second energy acceptor moieties,
wherein:
the fourth nucleotide sequence is complementary to the complement of the second nucleotide sequence of the first oligonucleotide,
the donor moiety is capable of emitting a first quantum of energy,
the first and second acceptor moieties are each capable of absorbing a substantial amount of the first quantum of energy,
the first acceptor moiety is capable of emitting a second quantum of energy,
the donor moiety is attached to a nucleotide of the first nucleotide sequence, the first acceptor moiety is attached to a nucleotide of the first nucleotide sequence, and the second acceptor moiety is attached to a nucleotide of the third nucleotide sequence; or the donor moiety is attached to a nucleotide of the third nucleotide sequence, the first acceptor moiety is attached to a nucleotide of the third nucleotide sequence, and the second acceptor moiety is attached to a nucleotide of the first nucleotide sequence,
the third oligonucleotide is capable of forming a hairpin containing a nucleotide of the first nucleotide sequence and a nucleotide of the third nucleotide sequence, and
if the donor moiety emits the first quantum of energy, then the first acceptor moiety absorbs a substantial amount of the emitted first quantum of energy if the hairpin is not formed; and the second acceptor moiety absorbs a substantial amount of the emitted first quantum of energy if the hairpin is formed;
(h) extending the 3' end of the third oligonucleotide using the extended second strand as a template to form a doubly extended first strand, wherein the doubly extended first strand is annealed to the extended second strand;
(i) separating the doubly extended first strand from the extended labeled second strand;
(j) annealing the second oligonucleotide to the doubly extended first strand;
(k) extending the 3' end of the second oligonucleotide using the doubly extended first strand as a template to form a doubly extended second strand,
wherein the doubly extended first strand is annealed to the doubly extended second strand;
(l) optionally amplifying the doubly extended first and second strands; and
(m) detecting a second quantum of energy emitted by the first acceptor moiety to detect the target nucleotide sequence.

32. The method of claim 31, wherein the first acceptor moiety absorbs the substantial amount of the emitted first quantum of energy only if the hairpin is not formed.

33. The method of claim 31, wherein the second acceptor moiety absorbs the substantial amount of the emitted first quantum of energy only if the hairpin is formed.

34. The method of any of claims 31 to 33, wherein step (1) comprises:
(1) separating the doubly extended first strand from the doubly extended second strand;
(2) annealing the second oligonucleotide to the doubly extended first strand, and annealing the third oligonucleotide to the doubly extended second strand;
(3) extending the 3' end of the second oligonucleotide using the doubly extended first strand as a template to form another doubly extended second strand,
wherein the doubly extended first strand is annealed to the other doubly extended second strand; and extending the 3' end of the third oligonucleotide using the doubly extended second strand as a template to form another doubly extended first strand, wherein the doubly extended second strand is annealed to the other doubly extended first strand; and
(4) repeating steps (1), (2) and (3) for a finite number of times, wherein, in step (1), the doubly extended first and second strands respectively are the doubly extended first strand and the other doubly extended second strand of step (3), or respectively are the other doubly extended first strand and the doubly extended second strand of step (3).

35. The method of any of claims 31 - 34, wherein the third nucleotide sequence is not complementary to the fourth nucleotide sequence.

36. The method of any of claims 31 - 34, wherein the third nucleotide sequence is complementary to the nucleotide sequence flanking the target nucleotide sequence.

37. The method of any of claims 31 - 36, wherein the donor moiety is fluorescein.

38. The method of any of claims 31 - 37, wherein the first acceptor moiety is ROX.

39. The method of any of claims 31 - 38, wherein the second acceptor moiety is DABSYL.

40. A method for determining if a first or second target nucleotide sequence is present in a sample comprising:
(a) contacting the sample with:
(1) a first oligonucleotide containing:
(i) a first nucleotide sequence;
(ii) a second nucleotide sequence at the 5' end of the first nucleotide sequence;
(iii) a third nucleotide sequence at the 5' end of the second nucleotide sequence; and
(iv) a molecular energy transfer trio including a first energy donor moiety, and first and second energy acceptor moieties,
wherein,
the first energy donor moiety is capable of emitting a first quantum of energy,
the first and second acceptor moieties are each capable of absorbing a substantial amount of the quantum of energy,
the first acceptor moiety is capable of emitting a third quantum of energy,
the first donor moiety is attached to a nucleotide of the first nucleotide sequence, the first acceptor moiety is attached to a nucleotide of the first nucleotide sequence, and the second acceptor moiety is attached to a nucleotide of the third nucleotide sequence; or the first donor moiety is attached to a nucleotide of the third nucleotide sequence, the first acceptor moiety is attached to a nucleotide of the third nucleotide sequence, and the second acceptor moiety is attached to a nucleotide of the first nucleotide sequence,
the first oligonucleotide is capable of forming a first hairpin containing a nucleotide of the first nucleotide sequence and a nucleotide of the third nucleotide sequence, and
if the first donor moiety emits the first quantum of energy, then the first acceptor moiety absorbs a substantial amount of the emitted first quantum of energy if the first hairpin is not formed, and the second acceptor moiety absorbs a substantial amount of the emitted first quantum of energy if the first hairpin is formed, and
(2) a second oligonucleotide containing:
(i) a fourth nucleotide sequence;
(ii) a fifth nucleotide sequence at the 5' end of the fourth nucleotide sequence;
(iii) a sixth nucleotide sequence at the 5' end of the fifth nucleotide sequence; and
(iv) a molecular energy transfer pair including a second energy donor moiety, and a third energy acceptor moiety,
wherein,
the second energy donor moiety is capable of emitting a second quantum of energy,
the third acceptor moiety is capable of absorbing a substantial amount of the second quantum of energy,
the third acceptor moiety is capable of emitting a fourth quantum of energy,
the second donor moiety is attached to a nucleotide of the fourth nucleotide sequence and the third acceptor moiety is attached to a nucleotide of the sixth nucleotide sequence; or the second donor moiety is attached to a nucleotide of the sixth nucleotide sequence and the third acceptor moiety is attached to a nucleotide of the fourth nucleotide sequence,
the second oligonucleotide is capable of forming a second hairpin containing a nucleotide of the fourth nucleotide sequence and a nucleotide of the sixth nucleotide sequence, and
if the second donor moiety emits the second quantum of energy, then the third acceptor moiety absorbs a substantial amount of the emitted second quantum of energy if the second hairpin is formed,
(b) incorporating:
(1) the first oligonucleotide into a first double-stranded nucleic acid if the first target nucleotide sequence is present in the sample, thereby preventing the hairpin of the first oligonucleotide from forming, and
(2) the second oligonucleotide into a second double-stranded nucleic acid if the second target nucleotide sequence is present in the sample, thereby preventing the hairpin of the second oligonucleotide from forming,
(c) optionally conducting:
(1) a first amplification reaction, thereby incorporating the first oligonucleotide into a first amplification product if the first target nucleotide sequence is present in the sample, and
(2) a second amplification reaction, thereby incorporating the second oligonucleotide into a second amplification product if the second target nucleotide sequence is present in the sample; and
(d) determining that:
(1) the first target nucleotide sequence is present in the sample if a third quantum of energy emitted by the first acceptor moiety is detected, or the first target nucleotide sequence is not present in the sample if the third quantum of energy is not detected, and
(2) the second target nucleotide sequence is present in the sample if a fourth quantum of energy emitted by the third acceptor moiety is detected, or the second target nucleotide sequence is not present in the sample if the fourth quantum of energy is not detected.

41. A method according to claim 40, wherein the first acceptor moiety absorbs a substantial amount of the emitted first quantum of energy only if the first hairpin is not formed.

42. A method of claim 40, wherein the second acceptor moiety absorbs a substantial amount of the emitted first quantum of energy only if the first hairpin is formed.

43. The method of any of claims 40 to 42, wherein the third acceptor moiety absorbs a substantial amount of the emitted second quantum of energy only if the second hairpin is formed.

44. The method of any of claims 40 to 43, wherein the first oligonucleotide further comprises a seventh nucleotide sequence at the 3' end of the first nucleotide sequence.

45. The method of claim 44, wherein the third nucleotide sequence is not complementary to the seventh nucleotide sequence.

46. The method of claim 44, wherein the seventh nucleotide sequence is complementary to a nucleotide sequence flanking the first target nucleotide sequence.

47. The method of any of claims 40 to 46, wherein the second oligonucleotide further comprises an eighth nucleotide sequence at the 3' end of the fourth nucleotide sequence.

48. The method of claim 47, wherein the sixth nucleotide sequence is not complementary to the eighth nucleotide sequence.

49. The method of claim 47, wherein the eighth nucleotide sequence is complementary to a nucleotide sequence flanking the second target nucleotide sequence.

50. The method according to any of claims 40 - 49, wherein the first and second donor moieties are each fluorescein.

51. The method according to any of claims 40 - 50, wherein the first acceptor moiety is ROX.

52. The method of any of claims 40 - 51, wherein the second and third acceptor moieties are each DABSYL.

53. The method of any of claims 40 - 52 wherein, in step (b)the first oligonucleotide is incorporated into the first double-stranded nucleic acids using a polymerase, and the second oligonucleotide is incorporated into the second double-stranded nucleic acid using a polymerase.

## Patentansprüche

1. Oligonucleotid mit:
(a) einer ersten Nucleotidsequenz;
(b) einer zweiten Nucleotidsequenz am 5'-Ende der ersten Nucleotidsequenz;
(c) einer dritten Nucleotidsequenz am 5'-Ende der zweiten Nucleotidsequenz; und
(d) einem molekularen Energietransfertrio, einschließlich eines Energiespenderanteils und eines ersten und zweiten Energieempfängeranteils,
wobei
der Energiespenderanteil ein Energiequantum abgeben kann und der erste und der zweite Empfängeranteil jeweils in der Lage sind, eine wesentliche Menge des Energiequantums: zu absorbieren,
der Spenderanteil an einem Nucleotid der ersten Nucleotidsequenz angefügt ist, der erste Empfängeranteil an einem Nucleotid der ersten Nucleotidsequenz angefügt ist, und der zweite Empfängeranteil an einem Nucleotid der dritten Nucleotidsequenz angefügt ist; oder der Spenderanteil an einem Nucleotid der dritten Nucleotidsequenz angefügt ist, der erste Empfängeranteil an einem Nucleotid der dritten Nucleotidsequenz angefügt ist, und der zweite Empfängeranteil an einem Nucleotid der ersten Nucleotidsequenz angefügt ist,
das Oligonucleotid eine Haarnadel bilden kann, die ein Nucleotid der ersten Nucleotidsequenz und ein Nucleotid der dritten Nucleotidsequenz enthält, und,
wenn der Spenderanteil das Energiequantum abgibt:
(1) der erste Empfängeranteil eine wesentliche Menge des abgegebenen Energiequantums absorbiert, wenn die Haarnadel nicht gebildet wird, und
(2) der zweite Empfängeranteil eine wesentliche Menge des abgegebenen Energiequantums absorbiert, wenn die Haarnadel gebildet wird.

2. Oligonucleotid nach Anspruch 1, wobei der erste Empfängeranteil die wesentliche Menge des abgegebenen Energiequantums nur dann absorbiert, wenn die Haarnadel nicht gebildet wird.

3. Oligonucleotid nach Anspruch 1, wobei der zweite Empfängeranteil die wesentliche Menge des abgegebenen Energiequantums nur dann absorbiert, wenn die Haarnadel gebildet wird.

4. Oligonucleotid nach einem der Ansprüche 1 bis 3, umfassend ein Desoxyribonucleotid.

5. Oligonucleotid nach einem der Ansprüche 1 bis 4, ferner umfassend eine vierte Nucleotidsequenz am 3'-Ende der ersten Nucleotidsequenz.

6. Oligonucleotid nach Anspruch 5, wobei die dritte Nucleotidsequenz nicht komplementär zur vierten Nucleotidsequenz ist.

7. Oligonucleotid nach Anspruch 5, wobei die vierte Nucleotidsequenz komplementär zur Nucleotidsequenz ist, die eine Zielnucleotidsequenz flankiert.

8. Oligonucleotid nach Anspruch 7, wobei die Zielnucleotidsequenz DNA ist.

9. Oligonucleotid nach einem der vorherigen Ansprüche, wobei der Spenderanteil ein Fluorophor ist.

10. Oligonucleotid nach Anspruch 9, wobei der zweite Empfängeranteil ein Quencher von Licht ist, das von dem Fluorophor abgegeben wird.

11. Oligonucleotid nach einem der vorherigen Ansprüche, wobei der erste Empfängeranteil noch ein zweites Energiequantum abgeben kann.

12. Oligonucleotid nach einem der vorherigen Ansprüche, wobei der erste Empfängeranteil ein Fluorophor ist.

13. Oligonucleotid nach einem der vorherigen Ansprüche, wobei der Spenderanteil Fluorescein ist.

14. Oligonucleotid nach einem der vorherigen Ansprüche, wobei der erste Empfängeranteil ROX ist.

15. Oligonucleotid nach einem der vorherigen Ansprüche, wobei der zweite Empfängeranteil DABSYL ist.

16. Oligonucleotid nach einem der vorherigen Ansprüche, wobei 0 bis 50 Nucleotide zwischen dem Nucleotid, an dem der Spenderanteil angefügt ist, und dem Nucleotid, an dem der erste Empfängeranteil angefügt ist, vorhanden sind.

17. Oligonucleotid nach Anspruch 16, wobei 5 bis 10 Nucleotide zwischen dem Nucleotid, an dem der Spenderanteil angefügt ist, und dem Nucleotid, an dem der erste Empfängeranteil angefügt ist, vorhanden sind.

18. Oligonucleotid nach einem der vorherigen Ansprüche, wobei 0 bis 50 Nucleotide zwischen dem Nucleotid, an dem der Spenderanteil angefügt ist, und dem Nucleotid, an dem der zweite Empfängeranteil angefügt ist, vorhanden sind.

19. Oligonucleotid nach Anspruch 18, wobei 5 bis 10 Nucleotide zwischen dem Nucleotid, an dem der Spenderanteil angefügt ist, und dem Nucleotid, an dem der zweite Empfängeranteil angefügt ist, vorhanden sind.

20. Oligonucleotid nach einem der vorherigen Ansprüche, wobei, wenn die Haarnadel gebildet wird, das Nucleotid, an dem der Spenderanteil angefügt ist, das Komplement des Nucleotids ist, an dem der zweite Empfängeranteil angefügt ist.

21. Oligonucleotid nach einem der vorherigen Ansprüche, wobei, wenn die Haarnadel gebildet wird, 0 bis 5 Nucleotide zwischen dem Nucleotid, an dem der Spenderanteil angefügt ist, und dem Komplement des Nucleotids, an dem der zweite Empfängeranteil angefügt ist, vorhanden sind.

22. Oligonucleotid, umfassend die Nucleotidsequenz SEQ ID Nr. 1, wobei Fluorescein an dem Nucleotid an Position 1 der SEQ ID Nr. 1 angefügt ist, ROX an dem Nucleotid an Position 21 der SEQ ID Nr. 1 angefügt ist, und DABSYL an dem Nucleotid an Position 5 oder 10 der SEQ ID Nr. 1 angefügt ist.

23. Oligonucleotid nach Anspruch 22, bestehend aus der Nucleotidsequenz SEQ ID Nr. 1.

24. Kit, umfassend das Oligonucleotid nach einem der vorherigenden Ansprüche und eine Polymerase.

25. Kit nach Anspruch 24, wobei die Polymerase eine DNA-Polymerase ist.

26. Verfahren zum Bestimmen, ob eine Zielnucleotidsequenz in einer Probe vorliegt, umfassend die folgenden Schritte:
(a) Inkontaktbringen der Probe mit einem Oligonucleotid nach einem der Ansprüche 11 bis 21 (in Abhängigkeit von Anspruch 11), 22 oder 23; und
(b) wenn das zweite Energiequantum nachgewiesen wird, Bestimmen, ob die Zielnucleotidsequenz in der Probe vorliegt; oder, wenn das zweite Energiequantum nicht nachgewiesen wird, Bestimmen, ob die Zielnucleotidsequenz nicht in der Probe vorliegt.

27. Verfahren zum Bestimmen, ob eine Zielnucleotidsequenz in einer Probe vorliegt, umfassend die folgenden Schritte:
(a) Inkontaktbringen der Probe mit einem Oligonucleotid nach einem der Ansprüche 11 bis 21 (in Abhängigkeit von Anspruch 11), 22 oder 23;
(b) Einbauen des Oligonucleotids in eine doppelsträngige Nucleinsäure, wenn die Zielnucleotidsequenz in der Probe vorliegt, so dass verhindert wird, dass die Haarnadel gebildet wird;
(c) bei Bedarf Durchführen einer Amplifikationsreaktion, so dass das Oligonucleotid in ein Amplifikationsprodukt eingebaut wird, wenn die Zielnucleotidsequenz in der Probe vorliegt; und
(d) wenn das zweite Energiequantum nachgewiesen wird, Bestimmen, ob die Zielnucleotidsequenz in der Probe vorliegt; oder, wenn das zweite Energiequantum nicht nachgewiesen wird, Bestimmen, ob die Zielnucleotidsequenz nicht in der Probe vorliegt.

28. Verfahren nach Anspruch 27, wobei in Schritt (b) das Oligonucleotid in die doppelsträngige Nucleinsäure unter Verwendung einer Polymerase eingebaut wird.

29. Verfahren für den Nachweis einer Zielnucleotidsequenz, umfassend die folgenden Schritte:
(a) Anlagern eines ersten Oligonucleotids an eine Nucleotidsequenz, die eine Zielnucleotidsequenz flankiert, wobei das erste Oligonucleotid eines gemäß einem der Ansprüche 11 bis 23 ist;
(b) Verlängern des 3'-Endes des ersten Oligonucleotids unter Verwendung der Zielnucleotidsequenz als Matrize, um einen verlängerten ersten Strang zu bilden, wobei die Zielnucleotidsequenz an den verlängerten ersten Strang angelagert wird;
(c) Trennen der Zielnucleotidsequenz von dem verlängerten ersten Strang;
(d) Anlagern eines zweiten Oligonucleotids an den verlängerten ersten Strang;
(e) Verlängern des 3'-Endes des zweiten Oligonucleotids unter Verwendung des verlängerten ersten Strangs als Matrize, um einen verlängerten zweiten Strang zu bilden, wobei der verlängerte erste Strang an den verlängerten zweiten Strang angelagert wird;
(f) bei Bedarf Amplifizieren des verlängerten ersten und zweiten Strangs; und
(g) Nachweisen eines zweiten Energiequantums, das von dem ersten Empfängeranteil abgegeben wird, um die Zielnucleotidsequenz nachzuweisen.

30. Verfahren nach Anspruch 29, wobei Schritt (f) Folgendes umfasst:
(1) Trennen des verlängerten ersten Strangs von dem verlängerten zweiten Strang;
(2) Anlagern des ersten Oligonucleotids an den verlängerten zweiten Strang und Anlagern des zweiten Oligonucleotids an den verlängerten ersten Strang;
(3) Verlängern des 3'-Endes des ersten Oligonucleotids unter Verwendung des verlängerten zweiten Strangs als Matrize, um einen weiteren verlängerten ersten Strang zu bilden, wobei der verlängerte zweite Strang an den anderen verlängerten ersten Strang angelagert wird; und Verlängern des 3'-Endes des zweiten Oligonucleotids unter Verwendung des verlängerten ersten Strangs als Matrize, um einen weiteren verlängerten zweiten Strang zu bilden, wobei der verlängerte erste Strang an den anderen verlängerten zweiten Strang angelagert wird; und
(4) Wiederholen der Schritte (1), (2) und (3) mit einer begrenzten Häufigkeit, wobei in Schritt (1) der verlängerte erste und zweite Strang jeweils der verlängerte erste Strang und der andere verlängerte zweite Strang aus Schritt (3) sind, oder jeweils der andere verlängerte erste Strang und der verlängerte zweite Strang aus Schritt (3) sind.

31. Verfahren für den Nachweis einer Zielnucleotidsequenz, umfassend die folgenden Schritte:
(a) Anlagern eines ersten Nucleotids an eine Nucleotidsequenz, die eine Zielnucleotidsequenz flankiert, wobei das erste Oligonucleotid Folgendes enthält:
(1) eine erste Nucleotidsequenz, die komplementär zu der Nucleotidsequenz ist, die die Zielnucleotidsequenz flankiert, und
(2) eine zweite Nucleotidsequenz am 5'-Ende der ersten Nucleotidsequenz;
(b) Verlängern des 3'-Endes des ersten Oligonucleotids unter Verwendung der Zielnucleotidsequenz als Matrize zur Bildung eines verlängerten ersten Strangs, wobei die Zielnucleotidsequenz an den verlängerten ersten Strang angelagert wird;
(c) Trennen der Zielnucleotidsequenz von dem verlängerten ersten Strang;
(d) Anlagern eines zweiten Oligonucleotids an den verlängerten ersten Strang;
(e) Verlängern des 3'-Endes des zweiten Oligonucleotids unter Verwendung des verlängerten ersten Strangs als Matrize zur Bildung eines verlängerten zweiten Strangs, wobei der verlängerte erste Strang an den verlängerten zweiten Strang angelagert wird;
(f) Trennen des verlängerten ersten Strangs von dem verlängerten zweiten Strang;
(g) Anlagern eines dritten Oligonucleotids an den verlängerten zweiten Strang, wobei das dritte Oligonucleotid Folgendes enthält:
(1) eine erste Nucleotidsequenz,
(2) eine zweite Nucleotidsequenz am 5'-Ende der ersten Nucleotidsequenz,
(3) eine dritte Nucleotidsequenz am 5'-Ende der zweiten Nucleotidsequenz,
(4) eine vierte Nucleotidsequenz am 3'-Ende der ersten Nucleotidsequenz, und
(5) ein molekulares Energietransfertrio, umfassend einen Energiespenderanteil und einen ersten und zweiten Energieempfängeranteil,
wobei:
die vierte Nucleotidsequenz komplementär zum Komplement der zweiten Nucleotidsequenz des ersten Oligonucleotids ist,
der Spenderanteil ein erstes Energiequantum abgeben kann,
der erste und der zweite Empfängeranteil jeweils in der Lage sind, eine wesentliche Menge des ersten Energiequantums zu absorbieren,
der erste Empfängeranteil ein zweites Energiequantum abgeben kann,
der Spenderanteil an einem Nucleotid der ersten Nucleotidsequenz angefügt ist, der erste Empfängeranteil an einem Nucleotid der ersten Nucleotidsequenz angefügt ist, und der zweite Empfängeranteil an einem Nucleotid der dritten Nucleotidsequenz angefügt ist; oder der Spenderanteil an einem Nucleotid der dritten Nucleotidsequenz angefügt ist, der erste Empfängeranteil an einem Nucleotid der dritten Nucleotidsequenz angefügt ist, und der zweite Empfängeranteil an einem Nucleotid der ersten Nucleotidsequenz angefügt ist,
das dritte Oligonucleotid eine Haarnadel bilden kann, die ein Nucleotid der ersten Nucleotidsequenz und ein Nucleotid der dritten Nucleotidsequenz enthält, und,
wenn der Spenderanteil das erste Energiequantum abgibt, der erste Empfängeranteil eine wesentliche Menge des abgegebenen ersten Energiequantums absorbiert, wenn die Haarnadel nicht gebildet wird; und der zweite Empfängeranteil eine wesentliche Menge des abgegebenen ersten Energiequantums absorbiert, wenn die Haarnadel gebildet wird;
(h) Verlängern des 3'-Endes des dritten Oligonucleotids unter Verwendung des verlängerten zweiten Strangs als Matrize, um einen doppelt verlängerten ersten Strang zu bilden, wobei der doppelt verlängerte erste Strang an den verlängerten zweiten Strang angelagert wird;
(i) Trennen des doppelt verlängerten ersten Strangs von dem verlängerten markierten zweiten Strang;
(j) Anlagern des zweiten Oligonucleotids an den doppelt verlängerten ersten Strang;
(k) Verlängern des 3'-Endes des zweiten Oligonucleotids unter Verwendung des doppelt verlängerten ersten Strangs als Matrize, um einen doppelt verlängerten zweiten Strang zu bilden, wobei der doppelt verlängerte erste Strang an den doppelt verlängerten zweiten Strang angelagert wird;
(l) bei Bedarf Amplifizieren des doppelt verlängerten ersten und zweiten Strangs; und
(m) Nachweisen eines zweiten Energiequantums, das von dem ersten Empfängeranteil abgegeben wird, um die Zielnucleotidsequenz nachzuweisen.

32. Verfahren nach Anspruch 31, wobei der erste Empfängeranteil die wesentliche Menge des abgegebenen ersten Energiequantums nur dann absorbiert, wenn die Haarnadel nicht gebildet wird.

33. Verfahren nach Anspruch 31, wobei der zweite Empfängeranteil die wesentliche Menge des abgegebenen ersten Energiequantums nur dann absorbiert, wenn die Haarnadel gebildet wird.

34. Verfahren nach einem der Ansprüche 31 bis 33, wobei Schritt (1) Folgendes umfasst:
(1) Trennen des doppelt verlängerten ersten Strangs von dem doppelt verlängerten zweiten Strang;
(2) Anlagern des zweiten Oligonucleotids am doppelt verlängerten ersten Strang und Anlagern des dritten Oligonucleotids am doppelt verlängerten zweiten Strang;
(3) Verlängern des 3'-Endes des zweiten Oligonucleotids unter Verwendung des doppelt verlängerten ersten Strangs als Matrize zur Bildung eines weiteren doppelt verlängerten zweiten Strangs, wobei der doppelt verlängerte erste Strang an den anderen doppelt verlängerten zweiten Strang angelagert wird; und Verlängern des 3'-Endes des dritten Oligonucleotids unter Verwendung des doppelt verlängerten zweiten Strangs als Matrize zur Bildung eines weiteren doppelt verlängerten ersten Strangs, wobei der doppelt verlängerte zweite Strang an dem anderen doppelt verlängerten ersten Strang angelagert wird; und
(4) Wiederholen der Schritte (1), (2) und (3) mit einer begrenzten Häufigkeit, wobei in Schritt (1) der doppelt verlängerte erste und zweite Strang jeweils der doppelt verlängerte erste Strang und der andere doppelt verlängerte zweite Strang aus Schritt (3) sind, oder jeweils der andere doppelt verlängerte erste Strang und der doppelt verlängerte zweite Strang aus Schritt (3) sind.

35. Verfahren nach einem der Ansprüche 31 - 34, wobei die dritte Nucleotidsequenz nicht komplementär zur vierten Nucleotidsequenz ist.

36. Verfahren nach einem der Ansprüche 31 - 34, wobei die dritte Nucleotidsequenz komplementär zur Nucleotidsequenz ist, die die Zielnucleotidsequenz flankiert.

37. Verfahren nach einem der Ansprüche 31 - 36, wobei der Spenderanteil Fluorescein ist.

38. Verfahren nach einem der Ansprüche 31 - 37, wobei der erste Empfängeranteil ROX ist.

39. Verfahren nach einem der Ansprüche 31 - 38, wobei der zweite Empfängeranteil DABSYL ist.

40. Verfahren zum Bestimmen, ob eine erste oder zweite Zielnucleotidsequenz in einer Probe vorliegt, umfassend die folgenden Schritte:
(a) Inkontaktbringen der Probe mit:
(1) einem ersten Oligonucleotid, umfassend:
(i) eine erste Nucleotidsequenz;
(ii) eine zweite Nucleotidsequenz am 5'-Ende der ersten Nucleotidsequenz;
(iii) eine dritte Nucleotidsequenz am 5'-Ende der zweiten Nucleotidsequenz; und
(iv) ein molekulares Energietransfertrio, einschließlich eines ersten Energiespenderanteils und eines ersten und zweiten Energieempfängeranteils,
wobei
der erste Energiespenderanteil ein erstes Energiequantum abgeben kann,
der erste und der zweite Empfängeranteil jeweils eine wesentliche Menge des Energiequantums absorbieren können,
der erste Empfängeranteil ein drittes Energiequantum abgeben kann,
der erste Spenderanteil an einem Nucleotid der ersten Nucleotidsequenz angefügt ist, der erste Empfängeranteil an einem Nucleotid der ersten Nucleotidsequenz angefügt ist, und der zweite Empfängeranteil an einem Nucleotid der dritten Nucleotidsequenz angefügt ist; oder der erste Spenderanteil an einem Nucleotid der dritten Nucleotidsequenz angefügt ist, der erste Empfängeranteil an einem Nucleotid der dritten Nucleotidsequenz angefügt ist, und der zweite Empfängeranteil an einem Nucleotid der ersten Nucleotidsequenz angefügt ist,
das erste Oligonucleotid eine erste Haarnadel bilden kann, die ein Nucleotid der ersten Nucleotidsequenz und ein Nucleotid der dritten Nucleotidsequenz enthält, und,
wenn der erste Spenderanteil das erste Energiequantum abgibt, der erste Empfängeranteil eine wesentliche Menge des abgegebenen ersten Energiequantums absorbiert, wenn die erste Haarnadel nicht gebildet wird, und der zweite Empfängeranteil eine wesentliche Menge des abgegebenen ersten Energiequantums absorbiert, wenn die erste Haarnadel gebildet wird, und
(2) einem zweiten Oligonucleotid, umfassend;
(i) eine vierte Nucleotidsequenz;
(ii) eine fünfte Nucleotidsequenz am 5'-Ende der vierten Nucleotidsequenz;
(iii) eine sechste Nucleotidsequenz am 5'-Ende der fünften Nucleotidsequenz; und
(iv) ein molekulares Energietransferpaar; einschließlich eines zweiten Energiespenderanteils und eines dritten Energieempfängeranteils,
wobei
der zweite Energiespenderanteil ein zweites Energiequantum abgeben kann,
der dritte Empfängeranteil eine wesentliche Menge des zweiten Energiequantums absorbieren kann,
der dritte Empfängeranteil ein viertes Energiequantum abgeben kann,
der zweite Spenderanteil an einem Nucleotid der vierten Nucleotidsequenz angefügt ist und der dritte Empfängeranteil an einem Nucleotid der sechsten Nucleotidsequenz angefügt ist; oder der zweite Spenderanteil an einem Nucleotid der sechsten Nucleotidsequenz angefügt ist und der dritte Empfängeranteil an einem Nucleotid der vierten Nucleotidsequenz angefügt ist,
das zweite Oligonucleotid eine zweite Haarnadel bilden kann, die ein Nucleotid der vierten Nucleotidsequenz und ein Nucleotid der sechsten Nucleotidsequenz enthält, und,
wenn der zweite Spenderanteil das zweite Energiequantum abgibt, der dritte Empfängeranteil eine wesentliche Menge des abgegebenen zweiten Energiequantums absorbiert, wenn die zweite Haarnadel gebildet wird,
(b) Einbauen:
(1) des ersten Oligonucleotids in eine erste doppelsträngige Nucleinsäure, wenn die erste Zielnucleotidsequenz in der Probe vorliegt, so dass verhindert wird, dass sich die Haarnadel des ersten Oligonucleotids bildet, und
(2) des zweiten Oligonucleotids in eine zweite doppelsträngige Nucleinsäure, wenn die zweite Zielnucleotidsequenz in der Probe vorliegt, so dass verhindert wird, dass sich die Haarnadel des zweiten Oligonucleotids bildet,
(c) bei Bedarf Durchführen:
(1) einer ersten Amplifikationsreaktion, wodurch das erste Oligonucleotid in ein erstes Amplifikationsprodukt eingebaut wird, wenn die erste Zielnucleotidsequenz in der Probe vorliegt, und
(2) einer zweiten Amplifikationsreaktion, wodurch das zweite Oligonucleotid in ein zweites Amplifikationsprodukt eingebaut wird, wenn die zweite Zielnucleotidsequenz in der Probe vorliegt; und
(d) Bestimmen, ob:
(1) die erste Zielnucleotidsequenz in der Probe vorliegt, wenn ein von dem ersten Empfängeranteil abgegebenes drittes Energiequantum nachgewiesen wird, oder die erste Zielnucleotidsequenz nicht in der Probe vorliegt, wenn das dritte Energiequantum nicht nachgewiesen wird, und
(2) die zweite Zielnucleotidsequenz in der Probe vorliegt, wenn ein von dem dritten Empfängeranteil abgegebenes viertes Energiequantum nachgewiesen wird, oder die zweite Zielnucleotidsequenz nicht in der Probe vorliegt, wenn das vierte Energiequantum nicht nachgewiesen wird.

41. Verfahren nach Anspruch 40, wobei der erste Empfängeranteil eine wesentliche Menge des abgegebenen ersten Energiequantums nur dann absorbiert, wenn die erste Haarnadel nicht gebildet wird.

42. Verfahren nach Anspruch 40, wobei der zweite Empfängeranteil eine wesentliche Menge des abgegebenen ersten Energiequantums nur dann absorbiert, wenn die erste Haarnadel gebildet wird.

43. Verfahren nach einem der Ansprüche 40 bis 42, wobei der dritte Empfängeranteil eine wesentliche Menge des abgegebenen zweiten Energiequantums nur dann absorbiert, wenn die zweite Haarnadel gebildet wird.

44. Verfahren nach einem der Ansprüche 40 bis 43, wobei das erste Oligonucleotid ferner eine siebte Nucleotidsequenz am 3'-Ende der ersten Nucleotidsequenz umfasst.

45. Verfahren nach Anspruch 44, wobei die dritte Nucleotidsequenz nicht komplementär zur siebten Nucleotidsequenz ist.

46. Verfahren nach Anspruch 44, wobei die siebte Nucleotidsequenz komplementär zu einer Nucleotidsequenz ist, die die erste Zielnucleotidsequenz flankiert.

47. Verfahren nach einem der Ansprüche 40 bis 46, wobei das zweite Oligonucleotid ferner eine achte Nucleotidsequenz am 3'-Ende der vierten Nucleotidsequenz umfasst.

48. Verfahren nach Anspruch 47, wobei die sechste Nucleotidsequenz nicht komplementär zur achten Nucleotidsequenz ist.

49. Verfahren nach Anspruch 47, wobei die achte Nucleotidsequenz komplementär zu einer Nucleotidsequenz ist, die die zweite Zielnucleotidsequenz flankiert.

50. Verfahren nach einem der Ansprüche 40 - 49, wobei der erste und der zweite Spenderanteil jeweils Fluorescein sind.

51. Verfahren nach einem der Ansprüche 40 - 50, wobei der erste Empfängeranteil ROX ist.

52. Verfahren nach einem der Ansprüche 40 - 51, wobei der zweite und der dritte Empfängeranteil jeweils DABSYL sind.

53. Verfahren nach einem der Ansprüche 40 - 52, wobei in Schritt (b) das erste Oligonucleotid in die ersten doppelsträngigen Nucleinsäuren unter Verwendung einer Polymerase eingebaut wird und das zweite Oligonucleotid in die zweite doppelsträngige Nucleinsäure unter Verwendung einer Polymerase eingebaut wird.

## Revendications

1. Oligonucléotide contenant :
(a) une première séquence nucléotidique;
(b) une deuxième séquence nucléotidique à l'extrémité 5' de la première séquence nucléotidique;
(c) une troisième séquence nucléotidique à l'extrémité 5' de la deuxième séquence nucléotidique; et
(d) un trio de transfert d'énergie moléculaire comprenant une moitié donneur d'énergie, et une première et une deuxième moitiés accepteurs d'énergie,
où
la moitié donneur d'énergie est capable d'émettre un quantum d'énergie, et la première et la deuxième moitiés accepteurs d'énergie sont chacune capables d'absorber une quantité substantielle du quantum d'énergie,
la moitié donneur est attachée à un nucléotide de la première séquence nucléotidique, la première moitié accepteur est attachée à un nucléotide de la première séquence nucléotidique, et la deuxième moitié accepteur est attachée à un nucléotide de la troisième séquence nucléotidique; ou bien la moitié donneur est attachée à un nucléotide de la troisième séquence nucléotidique, la première moitié accepteur est attachée à un nucléotide de la troisième séquence nucléotidique, et la deuxième moitié accepteur est attachée à un nucléotide de la première séquence nucléotidique,
l'oligonucléotide est capable de former une épingle à cheveux contenant un nucléotide de la première séquence nucléotidique et un nucléotide de la troisième séquence nucléotidique, et
si la moitié donneur émet le quantum d'énergie, alors :
(1) la première moitié accepteur absorbe une quantité substantielle du quantum émis d'énergie si l'épingle à cheveux n'est pas formée, et
(2) la deuxième moitié accepteur absorbe une quantité substantielle du quantum émis d'énergie si l'épingle à cheveux est formée.

2. L'oligonucléotide de la revendication 1, dans lequel la première moitié accepteur absorbe la quantité substantielle du quantum émis d'énergie seulement si l'épingle à cheveux n'est pas formée.

3. L'oligonucléotide de la revendication 1, dans lequel la deuxième moitié accepteur d'énergie absorbe la quantité substantielle du quantum émis d'énergie seulement si l'épingle à cheveux est formée.

4. L'oligonucléotide de l'une quelconque des revendications 1 à 3, comprenant un désoxyribonucléotide.

5. L'oligonucléotide de l'une quelconque des revendications 1 à 4, comprenant en outre une quatrième séquence nucléotidique à l'extrémité 3' de la première séquence nucléotidique.

6. L'oligonucléotide de la revendication 5, dans lequel la troisième séquence nucléotidique n'est pas complémentaire à la quatrième séquence nucléotidique.

7. L'oligonucléotide de la revendication 5, dans lequel la quatrième séquence nucléotidique est complémentaire à une séquence nucléotidique flanquant une séquence nucléotidique cible.

8. L'oligonucléotide de la revendication 7, dans lequel la séquence nucléotidique cible est de l'ADN.

9. L'oligonucléotide de l'une quelconque des revendications précédentes, dans lequel la moitié donneur est un fluorophore.

10. L'oligonucléotide de la revendication 9, dans lequel la deuxième moitié accepteur est un extincteur de lumière émise par le fluorophore.

11. L'oligonucléotide de l'une quelconque des revendications précédentes, dans lequel la première moitié accepteur est capable d'émettre un autre deuxième quantum d'énergie.

12. L'oligonucléotide de l'une quelconque des revendications précédentes, dans lequel la première moitié accepteur est un fluorophore.

13. L'oligonucléotide selon l'une quelconque des revendications précédentes, dans lequel la moitié donneur est de la fluorescéine.

14. L'oligonucléotide selon l'une quelconque des revendications précédentes, dans lequel la première moitié accepteur est de la ROX.

15. L'oligonucléotide selon l'une quelconque des revendications précédentes, dans lequel, la deuxième moitié accepteur est du DABSYL.

16. L'oligonucléotide selon l'une quelconque des revendications précédentes, dans lequel il y a 0 à 50 nucléotide(s) entre le nucléotide auquel la moitié donneur est attachée et le nucléotide auquel la première moitié accepteur est attachée.

17. L'oligonucléotide de la revendication 16, dans lequel il y a 5 à 10 nucléotides entre le nucléotide auquel la moitié donneur est attachée et le nucléotide auquel la première moitié accepteur est attachée.

18. L'oligonucléotide selon l'une quelconque des revendications précédentes, dans lequel il y a 0 à 50 nucléotide(s) entre le nucléotide auquel la moitié donneur est attachée et le nucléotide auquel la deuxième moitié accepteur est attachée.

19. L'oligonucléotide de la revendication 18, dans lequel il y a 5 à 10 nucléotides entre le nucléotide auquel la moitié donneur est attachée et le nucléotide auquel la deuxième moitié accepteur est attachée.

20. L'oligonucléotide de l'une quelconque des revendications précédentes, dans lequel, si l'épingle à cheveux est formée, alors le nucléotide auquel la moitié donneur est attachée est le complément du nucléotide auquel la deuxième moitié donneur est attachée.

21. L'oligonucléotide de l'une quelconque des revendications précédentes, dans lequel, si l'épingle à cheveux est formée, alors il y a 0 à 5 nucléotide(s) entre le nucléotide auquel la moitié donneur est attachée et le complément du nucléotide auquel la deuxième moitié accepteur est attachée.

22. Oligonucléotide comprenant la séquence nucléotidique de la SEQ ID NO:1, où la fluorescéine est attachée au nucléotide à la position 1 de la SEQ ID NO:1, la ROX est attachée au nucléotide à la position 21 de la SEQ ID NO:1, et le DABSYL est attaché au nucléotide à la position 5 ou 10 de la SEQ ID NO:1.

23. L'oligonucléotide de la revendication 22, consistant en la séquence nucléotidique de la SEQ ID NO:1.

24. Kit comprenant l'oligonucléotide de l'une quelconque des revendications précédentes et une polymérase.

25. Le kit de la revendication 24, dans lequel la polymérase est une ADN polymérase.

26. Méthode pour déterminer si une séquence nucléotidique cible est présente dans un échantillon, comprenant :
(a) mettre en contact l'échantillon avec un oligonucléotide de l'une quelconque des revendications 11 à 21 (lorsque dépendantes de la revendication 11), 22 ou 23; et
(b) si le deuxième quantum d'énergie est détecté, alors déterminer si la séquence nucléotidique cible est présente dans l'échantillon; ou si le deuxième quantum d'énergie n'est pas détectée, alors déterminer si la séquence nucléotidique cible n'est pas présente dans l'échantillon.

27. Méthode pour déterminer si une séquence nucléotidique cible est présente dans un échantillon, comprenant :
(a) mettre en contact l'échantillon avec un oligonucléotide de l'une quelconque des revendications 11 à 21 (lorsque dépendantes de la revendication 11), 22 ou 23;
(b) incorporer l'oligonucléotide dans un acide nucléique double brin si la séquence nucléotidique cible est présente dans l'échantillon, empêchant ainsi l'épingle à cheveux de se former;
(c) effectuer optionnellement une réaction d'amplification, incorporant ainsi l'oligonucléotide dans un produit d'amplification si la séquence nucléotidique cible est présente dans l'échantillon; et
(d) si le deuxième quantum d'énergie est détecté, alors déterminer si la séquence nucléotidique cible est présente dans l'échantillon; ou si le deuxième quantum d'énergie n'est pas détecté, alors déterminer si la séquence nucléotidique cible n'est pas présente dans l'échantillon.

28. La méthode de la revendication 27, dans laquelle à l'étape (b), l'oligonucléotide est incorporé dans l'acide nucléique double brin en utilisant une polymérase.

29. Méthode pour détecter une séquence nucléotidique cible comprenant :
(a) hybrider un premier oligonucléotide avec une séquence nucléotidique flanquant une séquence nucléotidique cible, où le premier oligonucléotide est un oligonucléotide selon l'une quelconque des revendications 11 à 23;
(b) prolonger l'extrémité 3' du premier oligonucléotide en utilisant la séquence nucléotidique cible comme une matrice pour former un premier brin prolongé, où la séquence nucléotidique cible est hybridée avec le premier brin;
(c) séparer la séquence nucléotidique cible du premier brin prolongé;
(d) hydrider un deuxième oligonucléotide au premier brin prolongé;
(e) prolonger l'extrémité 3' du deuxième oligonucléotide en utilisant le premier brin prolongé comme une matrice pour former un deuxième brin prolongé, où le premier brin prolongé est hybridé avec le deuxième brin prolongé;
(f) amplifier optionnellement les premier et deuxième brins prolongés; et
(g) détecter un deuxième quantum d'énergie émis par la première moitié accepteur pour détecter la séquence nucléotidique cible.

30. La méthode selon la revendication 29, dans laquelle l'étape (f) comprend :
(1) séparer le premier brin prolongé du deuxième brin prolongé;
(2) hybrider le premier oligonucléotide au deuxième brin prolongé et hybrider le deuxième oligonucléotide au premier brin prolongé;
(3) prolonger l'extrémité 3' du premier oligonucléotide en utilisant le deuxième brin prolongé comme une matrice pour former un autre premier brin prolongé, où le deuxième brin prolongé est hybridé avec l'autre premier brin prolongé; et prolonger l'extrémité 3' du deuxième oligonucléotide en utilisant le premier brin prolongé comme une matrice pour former un autre deuxième brin prolongé, où le premier brin prolongé est hybridé à l'autre deuxième brin prolongé; et
(4) répéter les étapes (1), (2) et (3) un nombre fini de fois, où, à l'étape (1), les premier et deuxième brins prolongés sont respectivement le premier brin prolongé et l'autre deuxième brin prolongé de l'étape (3), ou bien sont respectivement l'autre premier brin prolongé et le deuxième brin prolongé de l'étape (3).

31. Méthode pour détecter une séquence nucléotidique cible comprenant :
(a) hybrider un premier oligonucléotide avec une séquence nucléotidique flanquant une séquence nucléotidique cible, où le premier oligonucléotide contient :
(1) une première séquence nucléotidique complémentaire à la séquence nucléotidique flanquant la séquence nucléotidique cible; et
(2) une deuxième séquence nucléotidique à l'extrémité 5' de la première séquence nucléotidique.
(b) prolonger l'extrémité 3' du premier oligonucléotide en utilisant la séquence nucléotidique cible comme une matrice pour former un premier brin prolongé, où la séquence nucléotidique cible est hybridée avec le premier brin prolongé;
(c) séparer la séquence nucléotidique cible du premier brin prolongé;
(d) hybrider un deuxième oligonucléotide avec le premier brin prolongé;
(e) prolonger l'extrémité 3' du deuxième oligonucléotide en utilisant le premier brin prolongé comme une matrice pour former un deuxième brin prolongé, où le premier brin prolongé est hybridé avec le deuxième brin prolongé;
(f) séparer le premier brin prolongé du deuxième brin prolongé;
(g) hybrider un troisième oligonucléotide avec le deuxième brin prolongé, où le troisième oligonucléotide contient :
(1) une première séquence nucléotidique;
(2) une deuxième séquence nucléotidique à l'extrémité 5' de la première séquence nucléotidique,
(3) une troisième séquence nucléotidique à l'extrémité 5' de la deuxième séquence nucléotidique,
(4) une quatrième séquence nucléotidique à l'extrémité 3' de la première séquence nucléotidique, et
(5) un trio de transfert d'énergie moléculaire comprenant une moitié donneur d'énergie, et des première et deuxième moitiés accepteurs d'énergie,
où
la quatrième séquence nucléotidique est complémentaire au complément de la deuxième séquence nucléotidique du premier oligonucléotide,
la moitié donneur est capable d'émettre un premier quantum d'énergie,
les première et deuxième moitiés accepteurs sont chacune capables d'absorber une quantité substantielle du premier quantum d'énergie,
la première moitié accepteur est capable d'émettre un deuxième quantum d'énergie,
la moitié donneur est attaché à un nucléotide de la première séquence nucléotidique, la première moitié accepteur est attachée à un nucléotide de la première séquence nucléotidique, et la deuxième moitié accepteur est attachée à un nucléotide de la troisième séquence nucléotidique; ou bien la moitié donneur est attachée à un nucléotide de la troisième séquence nucléotidique, la première moitié accepteur est attachée à un nucléotide de la troisième séquence nucléotidique, et la deuxième moitié accepteur est attaché à un nucléotide de la première séquence nucléotidique,
le troisième nucléotide est capable de former une épingle à cheveux contenant un nucléotide de la première séquence nucléotidique et un nucléotide de la troisième séquence nucléotidique, et
si la moitié donneur émet le premier quantum d'énergie, alors la première moitié accepteur absorbe une quantité substantielle du premier quantum émis d'énergie si l'épingle à cheveux n'est pas formée; et la deuxième moitié accepteur absorbe une quantité substantielle du premier quantum émis d'énergie si l'épingle à cheveux est formée;
(h) prolonger l'extrémité 3' du troisième oligonucléotide en utilisant le deuxième brin prolongé comme une matrice pour former un premier brin doublement prolongé, où le premier brin doublement prolongé est hybridé avec le deuxième brin prolongé;
(i) séparer le premier brin doublement prolongé du deuxième brin prolongé marqué;
(j) hybrider le deuxième oligonucléotide avec le premier brin doublement prolongé;
(k) prolonger l'extrémité 3' du deuxième oligonucléotide en utilisant le premier brin doublement prolongé comme une matrice pour former un deuxième brin doublement prolongé, où le premier brin doublement prolongé est hybridé avec le deuxième brin doublement prolongé;
(1) amplifier optionnellement les premier et deuxième brins doublement prolongés; et
(m) détecter un deuxième quantum d'énergie émis par la première moitié accepteur pour détecter la séquence nucléotidique cible.

32. La méthode de la revendication 31, dans laquelle la première moitié accepteur absorbe la quantité substantielle du premier quantum émis d'énergie seulement si l'épingle à cheveux n'est pas formée.

33. La méthode de la revendication 31, dans laquelle la deuxième moitié accepteur absorbe la quantité substantielle du premier quantum émis d'énergie seulement si l'épingle à cheveux est formée.

34. La méthode de l'une quelconque des revendications 31 à 33, dans laquelle l'étape (1) comprend :
(1) séparer le premier brin doublement prolongé du deuxième brin doublement prolongé;
(2) hybrider le deuxième oligonucléotide au premier brin doublement prolongé et hybrider le troisième oligonucléotide au deuxième brin doublement prolongé;
(3) prolonger l'extrémité 3' du deuxième oligonucléotide en utilisant le premier brin doublement prolongé comme une matrice pour former un autre deuxième brin doublement prolongé, où le premier brin doublement prolongé est hybridé avec l'autre deuxième brin doublement prolongé; et prolonger l'extrémité 3' du troisième oligonucléotide en utilisant le deuxième brin doublement prolongé comme une matrice pour former un autre premier brin doublement prolongé, où le deuxième brin doublement prolongé est hybridé à l'autre premier brin doublement prolongé; et
(4) répéter les étapes (1), (2) et (3) un nombre fini de fois, où, à l'étape (1), les premier et deuxième brins doublement prolongés sont respectivement le premier brin doublement prolongé et l'autre deuxième brin doublement prolongé de l'étape (3), ou bien sont respectivement l'autre premier brin doublement prolongé et le deuxième brin doublement prolongé de l'étape (3).

35. La méthode de l'une quelconque des revendications 31 -34, dans laquelle la troisième séquence nucléotidique n'est pas complémentaire à la quatrième séquence nucléotidique.

36. La méthode de l'une quelconque des revendications 31 - 34, dans laquelle la troisième séquence nucléotidique est complémentaire à la séquence nucléotidique flanquant la séquence nucléotidique cible.

37. La méthode de l'une quelconque des revendications 31 - 36, dans laquelle la moitié donneur est de la fluorescéine.

38. La méthode de l'une quelconque des revendications 31 - 37, dans laquelle la première moitié accepteur est de la ROX.

39. La méthode de l'une quelconque des revendications 31 - 38, dans laquelle la deuxième moitié accepteur est du DABSYL.

40. Méthode pour déterminer si une première ou une deuxième séquence nucléotidique cible est présente dans un échantillon comprenant :
(a) mettre en contact l'échantillon avec :
(1) un premier oligonucléotide comprenant :
(i) une première séquence nucléotidique;
(ii) une deuxième séquence nucléotidique à l'extrémité 5' de la première séquence nucléotidique;
(iii) une troisième séquence nucléotidique à l'extrémité 5' de la deuxième séquence nucléotidique; et
(iv) un trio de transfert d'énergie moléculaire comprenant une première moitié donneur d'énergie, et une première et une deuxième moitiés accepteurs d'énergie,
où
la première moitié donneur d'énergie est capable d'émettre un premier quantum d'énergie,
la première et la deuxième moitiés accepteurs d'énergie sont chacune capables d'absorber une quantité substantielle du quantum d'énergie,
la première moitié accepteur est capable d'émettre un troisième quantum d'énergie,
la première moitié donneur est attachée à un nucléotide de la première séquence nucléotidique, la première moitié accepteur est attachée à un nucléotide de la première séquence nucléotidique, et la deuxième moitié accepteur est attachée à un nucléotide de la troisième séquence nucléotidique; ou bien la première moitié donneur est attachée à un nucléotide de la troisième séquence nucléotidique, la première moitié accepteur est attachée à un nucléotide de la troisième séquence nucléotidique, et la deuxième moitié accepteur est attachée à un nucléotide de la première séquence nucléotidique,
le premier oligonucléotide est capable de former une première épingle à cheveux contenant un nucléotide de la première séquence nucléotidique et un nucléotide de la troisième séquence nucléotidique, et
si la première moitié donneur émet le premier quantum d'énergie, alors la première moitié accepteur absorbe une quantité substantielle du premier quantum émis d'énergie si la première épingle à cheveux n'est pas formée, et la deuxième moitié accepteur absorbe une quantité substantielle du premier quantum émis d'énergie si la première épingle à cheveux est formée, et
(2) un deuxième oligonucléotide comprenant :
(i) une quatrième séquence nucléotidique;
(ii) une cinquième séquence nucléotidique à l'extrémité 5' de la quatrième séquence nucléotidique;
(iii) une sixième séquence nucléotidique à l'extrémité 5' de la cinquième séquence nucléotidique; et
(iv) un trio de transfert d'énergie moléculaire comprenant une deuxième moitié donneur d'énergie, et une troisième moitié accepteur d'énergie,
où
la deuxième moitié donneur d'énergie est capable d'émettre un deuxième quantum d'énergie,
la troisième moitié accepteur d'énergie est capable d'émettre une quantité substantielle du deuxième quantum d'énergie,
la troisième moitié accepteur est capable d'émettre un quatrième quantum d'énergie,
la deuxième moitié donneur est attachée à un nucléotide de la quatrième séquence nucléotidique et la troisième moitié accepteur est attachée à un nucléotide de la sixième séquence nucléotidique; ou bien la deuxième moitié donneur est attachée à un nucléotide de la sixième séquence nucléotidique et la troisième moitié accepteur est attachée à un nucléotide de la quatrième séquence nucléotidique,
le deuxième oligonucléotide est capable de former une deuxième épingle à cheveux contenant un nucléotide de la quatrième séquence nucléotidique et un nucléotide de la sixième séquence nucléotidique, et
si la deuxième moitié donneur émet le deuxième quantum d'énergie, alors la troisième moitié accepteur absorbe une quantité substantielle du deuxième quantum émis d'énergie si la première épingle à cheveux est formée,
(b) incorporer :
(1) le premier oligonucléotide dans un premier acide nucléique double brin si la première séquence nucléotidique cible est présente dans l'échantillon, empêchant ainsi l'épingle à cheveux du premier oligonucléotide de se former, et
(2) le deuxième oligonucléotide dans un deuxième acide nucléique double brin si la deuxième séquence nucléotidique cible est présente dans l'échantillon, empêchant ainsi l'épingle à cheveux du deuxième oligonucléotide de se former,
(c) effectuer optionnellement :
(1) une première réaction d'amplification, incorporant ainsi le premier oligonucléotide dans un premier produit d'amplification si la première séquence nucléotidique cible est présente dans l'échantillon, et
(2) une deuxième réaction d'amplification, incorporant ainsi le deuxième oligonucléotide dans un deuxième produit d'amplification si la deuxième séquence nucléotidique cible est présente dans l'échantillon; et
(d) déterminer que :
(1) la première séquence nucléotidique cible est présente dans l'échantillon si un troisième quantum d'énergie émis par la première moitié accepteur est détecté, ou bien que la première séquence nucléotidique cible n'est pas présente dans l'échantillon si le troisième quantum d'énergie n'est pas détecté, et
(2) la deuxième séquence nucléotidique cible est présente dans l'échantillon si un quatrième quantum d'énergie émis par la troisième moitié accepteur est détecté, ou bien que la deuxième séquence nucléotidique cible n'est pas présente dans l'échantillon si le quatrième quantum d'énergie n'est pas détecté.

41. Méthode selon la revendication 40, dans laquelle la première moitié accepteur absorbe une quantité substantielle du premier quantum émis d'énergie seulement si la première épingle à cheveux n'est pas formée.

42. Méthode de la revendication 40, dans laquelle la deuxième moitié accepteur absorbe une quantité substantielle du premier quantum émis d'énergie seulement si la première épingle à cheveux est formée.

43. Méthode de l'une quelconque des revendications 40 à 42, dans laquelle la troisième moitié accepteur absorbe une quantité substantielle du deuxième quantum émis d'énergie seulement si la deuxième épingle à cheveux est formée.

44. La méthode de l'une quelconque des revendications 40 à 43, dans laquelle le premier oligonucléotide comprend en outre une septième séquence nucléotidique à l'extrémité 3' de la première séquence nucléotidique.

45. La méthode de la revendication 44, dans laquelle la troisième séquence nucléotidique n'est pas complémentaire à la septième séquence nucléotidique.

46. La méthode de la revendication 44, dans laquelle la septième séquence nucléotidique est complémentaire à une séquence nucléotidique flanquant la première séquence nucléotidique cible.

47. La méthode de l'une quelconque des revendications 40 à 46, dans laquelle le deuxième oligonucléotide comprend en outre une huitième séquence nucléotidique à l'extrémité 3' de la quatrième séquence nucléotidique.

48. La méthode de la revendication 47, dans laquelle la sixième séquence nucléotidique n'est pas complémentaire à la huitième séquence nucléotidique.

49. La méthode de la revendication 47, dans laquelle la huitième séquence nucléotidique est complémentaire à une séquence nucléotidique flanquant la deuxième séquence nucléotidique cible.

50. La méthode selon l'une quelconque des revendications 40 - 49, dans laquelle les première et deuxième moitiés donneurs sont chacune de la fluorescéine.

51. La méthode selon l'une quelconque des revendications 40 - 50, dans laquelle la première moitié accepteur est de la ROX.

52. La méthode de l'une quelconque des revendications 40 - 51, dans laquelle les deuxième et troisième moitiés accepteurs sont chacune du DABSYL.

53. La méthode de l'une quelconque des revendications 40 - 52, dans laquelle, à l'étape (b), le premier oligonucléotide est incorporé dans les premiers acides nucléiques double brin en utilisant une polymérase, et le deuxième oligonucléotide est incorporé dans le deuxième acide nucléique double brin en utilisant une polymérase.
